(19) Europäisches Patentamt / European Patent Office / Office européen des brevets

(11) **EP 3 375 430 A1**

(12) **EUROPEAN PATENT APPLICATION**

(43) Date of publication:
19.09.2018 Bulletin 2018/38

(51) Int Cl.:
*A61K 8/34* (2006.01)     *A61Q 13/00* (2006.01)

(21) Application number: 18305289.3

(22) Date of filing: 16.03.2018

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **17.03.2017 EP 17305296**

(71) Applicant: **Takasago International Corporation Tokyo 144-8721 (JP)**

(72) Inventors:
• **RENOUD, Barbara**
**75017 PARIS (FR)**
• **BASSEREAU, Maud**
**75017 PARIS (FR)**
• **FRASER, Stuart**
**LITTLE NESTON, Cheshire CH64 4DH (GB)**

(74) Representative: **Cabinet Beau de Loménie 158, rue de l'Université 75340 Paris Cedex 07 (FR)**

(54) **FRAGRANCE COMPOSITIONS**

(57)     The invention relates to a long-lasting, ethanol-based fragrance composition. The composition comprises a fragrance, a diol and a surfactant in addition to ethanol.

EP 3 375 430 A1

Printed by Jouve, 75001 PARIS (FR)

**Description**

**Field of the Invention**

This invention relates to fragrance compositions for leave on cosmetic use which have greater fragrance retention on skin and other substrates than conventional alcoholic fragrances.

**Background**

Liquid fragrance compositions such as colognes, eau de parfum, eau de toilette, aftershave, body sprays and deodorants tend to be solutions of synthetic and natural fragrance ingredients in water alcohol mixtures. The alcohol has both negative and positive aspects in the formulation. On the positive side it gives a cooling tingling effect when perfume is applied which consumers associate with the application of perfume. On the negative side ethanol is flammable with a low flashpoint, its use is regulated in some countries and it can be an irritant particularly to the eyes. Thus replacing ethanol in fragrance products totally or especially in part can be beneficial.

There has always been an interest in providing a longer lasting fragrance by slowing the evaporation of fragrance ingredients from skin and other substrates. Traditionally this has been done by adding fixatives to the fragrance composition. Fixatives are fragrance ingredients which act as solvents for other fragrance ingredient slowing their evaporation. Generally, they are low volatility, hydrophobic compounds often with little odour. Classic fixatives used in fine fragrance are botanical absolutes, concretes, and resins or some animalic secretions such as ambergris. Synthetic fixatives are also known as are other methods for modifying fragrance evaporation as will be illustrated below.

US4264478 describes the use of alkoxylated methyl glucoside as a water miscible fixative and emollient which reduces the sting of alcohol.

US2383517 describes the use of an oleoresin extracted from *Myrica Asplenifolia* as a perfume fixative.

US6172037 describes a fixative comprising a combination of polyvinylpyrrolidone, hydroxypropyl cellulose and a hydrophobic oil.

EP 181401 describes ways of fixing perfume to substrates by gelling the fragrance in order to slow evaporation by slowing diffusion.

WO 2005/070371 describes a fragrance fixing complex comprising an hydrophobic, alcohol soluble, carboxylated acrylates octylacrylamide copolymer and a hydrolysed jojoba ester.

Thus known methods of creating a longer lasting fragrance within a conventional aqueous alcohol composition seem to depend on altering evaporation rates by adding hydrophobic materials to the fragrance, or by modifying fragrance evaporation by creating films or gels through which the fragrance has to diffuse in order to evaporate and be perceived, or by some combination of the above.

Often however the amount of hydrophobic material that can be added is limited either by the need to avoid a feeling of stickiness on the skin or of an odour associated with the fixative becoming noticeable if used at too high a level.

It is therefore desired to design an ethanol-based fragrance which retain top notes as well as middle notes for a long period of time when applied to skin, while being devoid of drawbacks such as stickiness or adverse sensory effects caused by an excessive amount of perfume fixatives.

**Summary of the Invention**

The present invention is directed to an ethanol-based fragrance composition which provides a longer lasting fragrance on skin, said composition comprising, based on the weight of the composition:

> a) about 1.0 to about 30.0 wt% fragrance;
> b) about 2.0 to about 30.0 wt% of a system comprised of a diol and a surfactant;
> c) about 25.0 to about 80.0 wt% ethanol;
> d) at least about 5.0 % water;

wherein the fragrance and the system (diol + surfactant) together make up at most 60.0 wt% of the composition;
wherein water and ethanol together make up at most 97.0 wt% of the composition;
wherein the surfactant is not a phospholipid.

In one embodiment, the surfactant is selected from anionic surfactants, zwitterionic surfactants, amphoteric surfactants, cationic surfactants, nonionic surfactants and mixtures thereof. The surfactant:fragrance weight ratio is preferably in the range from about 5:1 to about 0.5:1.

In one embodiment the diol is selected from vicinal diols, non-vicinal diols and mixtures thereof. The diol is preferably a non-vicinal diol. The surfactant:diol weight ratio is preferably in the range from about 4:1 to about 0.5:1.

In one embodiment, the (diol + surfactant):fragrance weight ratio is in the range from about 5:1 to about 0.8:1.

**Brief description of the figures**

Figures 1 to 4 show the headspace concentration of a control fragrance and of a fragrance composition of the invention just after application, 1h after application, 4h after application and 8h after application, of the respective fragrances on a blotter.

Figures 5 to 7 show the headspace concentration of a control fragrance and of a fragrance composition of the invention just after application, 4h after application and 8h after application, of the respective fragrances on a blotter.

Figures 8 to 10 show the headspace concentration of a control fragrance and of a fragrance composition of the invention just after application, 4h after application and 8h after application, of the respective fragrances on a blotter.

Figures 11 to 14 show the olfactive mapping of a control fragrance and of a fragrance of the invention just after application, 2h after application, 4h after application and 6h after application, of the respective fragrances on a human subject.

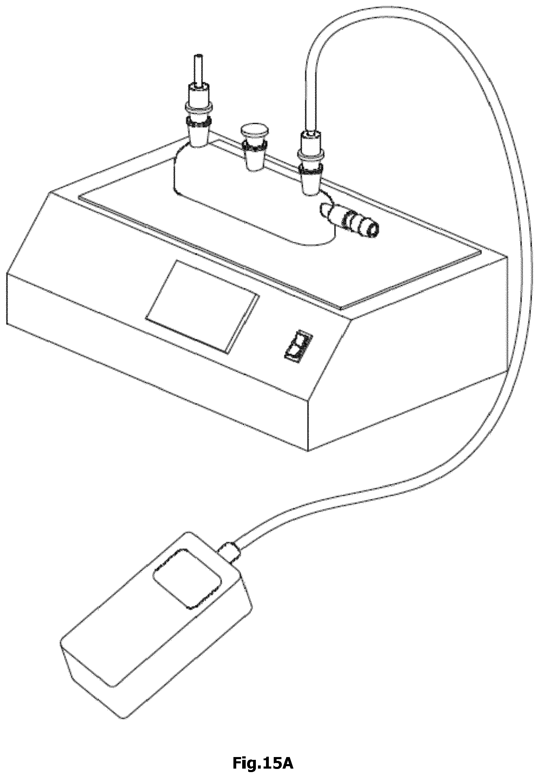

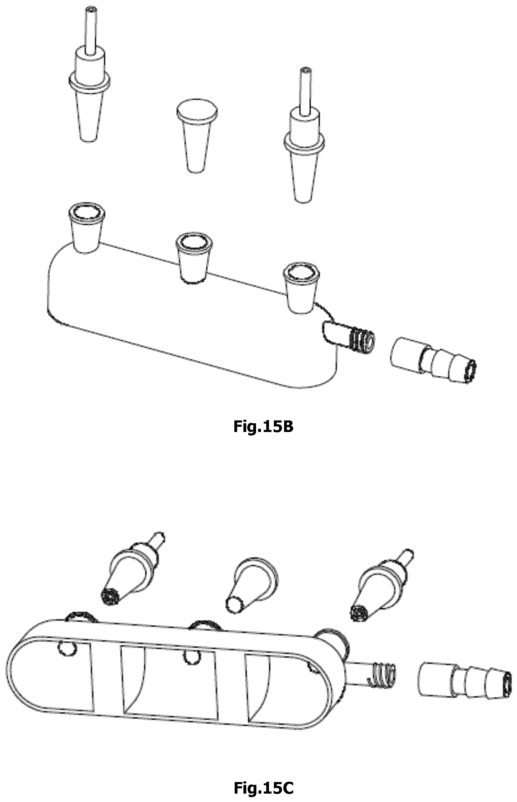

Figures 15A-15C show a headspace apparatus used in the invention.

Figures 16 to 21 show the headspace concentration of a control fragrance and of fragrance compositions of the invention just after application, 4h after application and 8h after application, of the respective fragrances on a blotter.

**Detailed Description of the Invention**

All percentages are quoted as weight percentages of the finished, consumable, product, i.e. excluding any packaging. Examples of packaging include, but are not limited to: bottles, trigger sprays, pump action sprays and pressurised aerosols etc.

The fragrance composition of the invention comprises:

a) about 1.0 to about 30.0 wt% fragrance;
b) about 2.0 to about 30.0 wt % of a system comprised of a diol and a surfactant;
c) about 25.0 to about 80.0 wt% ethanol;
d) at least about 5.0 wt% water;

wherein the fragrance and the system (diol + surfactant) together make up at most 60.0 wt% of the composition;

wherein water and ethanol together make up at most 97.0 wt% of the composition; wherein the surfactant is not a phospholipid.

**Fragrance composition**

The fragrance composition of the invention comprises from about 1.0 wt% to about 30.0 wt% fragrance.

According to the present invention, the term "fragrance" (also named "perfume") means any mixture of at least two fragrance ingredients (also named "fragrance materials"). A wide variety of odiferous materials are known for perfumery use, including compounds such as alkenes, alcohols, aldehydes, ketones, esters, ethers, nitriles, amines, oximes, acetals, ketals, thiols, thioketones, imines, etc. Without limiting the scope of the invention, the fragrance ingredients used in the fragrance preferably have a molecular weight of less than 375 atomic mass units (1 amu = 1 g/mol), preferably less than 325 amu, more preferably less than 300 amu, and even more preferably less than 275 amu, to ensure sufficient volatility to be noticeable. Furthermore, the fragrance ingredients preferably have a molecular weight greater than 100 amu, more preferably greater than 120 amu as lower masses may be too volatile or too water-soluble. The fragrance ingredients will not contain strongly ionizing functional groups such as sulphonates, sulphates, phosphates or quaternary ammonium ions.

Naturally occurring plant and animal oils, extracts, exudates and distillates, usually referred to as essential oils, comprise complex mixtures of various compounds and are also known for use as fragrance ingredients. Such ingredients can be used in the fragrance of the invention. Descriptions of many essential oils and methods of extraction and purification can be found in "The Essential Oils" by Ernest Guenther published by D. Van Nostrand in 1948, and may include extracts, pressings, the collection of exudates, and distillates from any part of suitable plants: roots, rhizomes, bulbs, corms, stem, bark, heartwood, leaves, flowers, seeds and fruit. Examples of such extracts and distillates include citrus fruit oils such as orange, mandarin, grapefruit, lime or lemon oils, tree oils such as pine, eucalyptus or cedarwood, herb oils such as peppermint, thyme, lavender, basil, rosemary, clove or flower extracts such as rose, jasmine, lily, or geranium oil. As is normal practise in fragrance formulation, the often complex mixtures that are essential oils will be considered as single ingredients when used in fragrances of the invention.

Fragrances of the invention can be relatively simple in their composition with a minimum of two fragrance ingredients or can comprise highly complex mixtures of natural and synthetic compounds, chosen to provide any desired odour. It is preferred if the fragrance composition contains more than five ingredients, more preferable that they contain more than eight ingredients, and even more preferable that they contain more than twelve ingredients. Fragrance ingredients are described more fully in S. Arctander, Perfume Flavors and Chemicals, Vols. I and II, Montclair, N. J., in the Merck Index, 8th Edition, Merck & Co., Inc. Rahway, N.J., and in Allured's FFM (Flavor and Fragrance Materials), all of which are incorporated herein by reference.

Advantageously, fragrance ingredients are selected from the following list:

- $C_8$-$C_{18}$ hydrocarbons, preferably delta-3-carene, alpha-pinene, beta-pinene, alphaterpinene, gamma-terpinene, p-cymene, bisabolene, camphene, caryophyllene, cedrene, farnesene, limonene, longifolene, myrcene, ocimene, valencene, (E,Z)-1,3,5-undecatriene;
- $C_2$-$C_{18}$ aliphatic alcohols, preferably hexanol, octanol, 3-octanol, 2,6-dimethylheptanol, 2-methylheptanol, 2-methyloctanol, (E)-3-hexenol, (E) and (Z)-3-hexenol, 1-octen-3-ol, mixtures of 3,4,5,6,6-pentamethyl-3/4-hepten-2-ol and 3,5,6,6-tetramethyl-4-methyleneheptan-2-ol, (E,Z)-2,6-nonadienol, 3,7-dimethyl-7-methoxyoctan-2-ol, 9-decenol, 10-undecenol, 4-methyl-3-decen-5-ol;
- $C_2$-$C_{18}$ aliphatic aldehydes and their acetals, preferably hexanal, heptanal, octanal, nonanal, decanal, undecanal, dodecanal, tridecanal, 2-methyloctanal, 2-methylnonanal, (E)-2-hexenal, (Z)-4-heptenal, 2,6-dimethyl-5-heptenal, 10-undecenal, (E)-4-decenal, 2-dodecenal, 2,6,10-trimethyl-5,9-undecadienal, heptanal diethyl acetal, 1,1-dimethoxy-2,2,5-trimethyl-4-hexene, citronellyl oxyacetaldehyde;
- $C_3$-$C_{18}$ aliphatic ketones and oximes thereof, preferably 2-heptanone, 2-octanone, 3-octanone, 2-nonanone, 5-methyl-3-heptanone, 5-methyl-3-heptanone oxime, 2,4,4,7-tetramethyl-6-octen-3-one;
- $C_2$-$C_{18}$ aliphatic sulphur-containing compounds, preferably 3-methylthiohexanol, 3-methylthiohexyl acetate, 3-mercaptohexanol, 3-mercaptohexyl acetate, 3-mercaptohexyl butyrate, 3-acetylthiohexyl acetate, 1-menthene-8-thiol;
- $C_2$-$C_{18}$ aliphatic nitrile-containing compounds, preferably 2-nonenenitrile, 2-tridecenenenitrile, 2,12-tridecenenenitrile, 3,7-dimethyl-2,6-octadienenitrile, 3,7-dimethyl-6-octenenitrile;
- $C_2$-$C_{18}$ aliphatic carboxylic acids and esters thereof, preferably (E)- and (Z)-3-hexenyl formate, ethyl acetoacetate, isoamyl acetate, hexyl acetate, 3,5,5-trimethylhexyl acetate, 3-methyl-2-butenyl acetate, (E)-2-hexenyl acetate, (E)- and (Z)-3-hexenyl acetate, octyl acetate, 3-octyl acetate, 1-octen-3-yl acetate, ethyl butyrate, butyl butyrate, isoamyl butyrate, hexyl butyrate, (E)- and (Z)-3-hexenyl isobutyrate, hexyl crotonate, ethyl isovalerate, ethyl 2-methylpentanoate, ethyl hexanoate, allyl hexanoate, ethyl heptanoate, allyl heptanoate, ethyl octanoate, ethyl (E,Z)-2,4-decadienoate, methyl 2-octynoate, methyl 2-nonynoate, allyl-2-isoamyloxyacetate, methyl-3,7-dimethyl-2,6-octadienoate;
- $C_4$-$C_{18}$ acyclic terpene alcohols, preferably citronellol, geraniol, nerol, linalool, lavandulol, nerolidol, farnesol, tetrahydrolinalool, tetrahydrogeraniol, 2,6-dimethyl-7-octen-2-ol, 2,6-dimethyloctan-2-ol, 2-methyl-6-methylene-7-octen-2-ol, 2,6-dimethyl-5,7-octadien-2-ol, 2,6-dimethyl-3,5-octadien-2-ol, 3,7-dimethyl-4,6-octadien-3-ol, 3,7-dimethyl-1,5,7-octatrien-3-ol, 2,6-dimethyl-2,5,7-octatrien-1-ol;
- $C_4$-$C_{18}$ acyclic terpene aldehydes and ketones, preferably geranial, neral, citronellal, 7-hydroxy-3,7-dimethyloctanal, 7-methoxy-3,7-dimethyloctanal, 2,6,10-trimethyl-9-undecenal, geranylacetone, and the dimethyl and diethyl acetals of geranial, neral, 7-hydroxy-3,7-dimethyloctanal;
- $C_4$-$C_{18}$ cyclic terpene alcohols, preferably alpha-terpineol, terpineol-4, menthan-8-ol, menthan-1-ol, menthan-7-ol, borneol, isoborneol, linalool oxide, nopol, cedrol, ambrinol, vetiverol, guaiol;
- $C_4$-$C_{18}$ cyclic terpene aldehydes and ketones, preferably fenchone, alpha-ionone, beta-ionone, alpha-n-methylionone, beta-n-methylionone, alpha-isomethylionone, beta-isomethylionone, alpha-irone, alpha-damascone, beta-damascone, beta-damascenone, delta-damascone, gamma-damascone, 1-(2,4,4-trimethyl-2-cyclohexen-1-yl)-2-buten-1-one, 1,3,4,6,7,8a-hexahydro-1,1,5,5-tetramethyl-2H-2,4a-methanonaphthalen-8(5H)-one, nootkatone, dihydronootkatone, alpha-sinensal, beta-sinensal, methyl cedryl ketone;
- $C_4$-$C_{18}$ cyclic alcohols, preferably 4-tert-butylcyclohexanol, 3,3,5-trimethylcyclohexanol, 3-isocamphylcyclohexanol, 2,6,9-trimethyl-Z2,Z5,E9-cyclododecatrien-1-ol, 2-isobutyl-4-methyltetrahydro-2H-pyran-4-ol;
- $C_4$-$C_{18}$ cycloaliphatic alcohols, preferably alpha-3,3-trimethylcyclohexylmethanol, 2-methyl-4-(2,2,3-trimethyl-3-cyclopent-1-yl)butanol, 2-methyl-4-(2,2,3-trimethyl-3-cyclopent-1-yl)-2-buten-1-ol, 2-ethyl-4-(2,2,3-trimethyl-3-cyclopent-1-yl)-2-buten-1-ol, 3-methyl-5-(2,2,3-trimethyl-3-cyclopent-1-yl)-pentan-2-ol, 3-methyl-5-(2,2,3-trimethyl-3-cyclopent-1-yl)-4-penten-2-ol, 3,3-dimethyl-5-(2,2,3-trimethyl-3-cyclopent-1-yl)-4-penten-2-ol, 1-(2,2,6-trimethylcyclohexyl)pentan-3-ol, 1-(2,2,6-trimethylcyclohexyl)hexan-3-ol;
- $C_4$-$C_{18}$ cyclic and cycloaliphatic ethers, preferably cedryl methyl ether, cyclododecyl methyl ether, (ethoxymethoxy)cyclododecane, alpha-cedrene epoxide, 3a,6,6,9a-tetramethyl-dodecahydronaphtho[2,1-b]furan, 3a-ethyl-6,6,9a-trimethyldodecahydro-naphtho[2,1-b]furan, 1,5,9-trimethyl-13-oxabicyclo[10.1.0]trideca-4,8-diene, rose oxide, 2-(2,4-dimethyl-3-cyclohexen-1-yl)-5-methyl-5-(1-methylpropyl)-1,3-dioxane;

- $C_4$-$C_{18}$ cyclic ketones, preferably 4-tert-butylcyclohexanone, 2,2,5-trimethyl-5-pentylcyclopentanone, 2-heptylcyclopentanone, 2-pentylcyclopentanone, 2-hydroxy-3-methyl-2-cyclopenten-1-one, 3-methyl-cis-2-penten-1-yl-2-cyclopenten-1-one, 3-methyl-2-pentyl-2-cyclopenten-1-one, 3-methyl-4-cyclopentadecenone, 3-methyl-5-cyclopentadecenone, 3-methylcyclopentadecanone, 4-(1-ethoxyvinyl)-3,3,5,5-tetramethylcyclohexanone, 4-tert-pentylcyclohexanone, 5-cyclohexadecen-1-one, 6,7-dihydro-1,1,2,3,3-pentamethyl-4(5H)-indanone, 9-cycloheptadecen-1-one, cyclopentadecanone, cyclohexadecanone;
- $C_4$-$C_{18}$ cycloaliphatic aldehydes, preferably 2,4-dimethyl-3-cyclohexenecarbaldehyde, 2-methyl-4-(2,2,6-trimethyl-cyclohexen-1-yl)-2-butenal, 4-(4-hydroxy-4-methylpentyl)-3-cyclohexenecarbaldehyde, 4-(4-methyl-3-penten-1-yl)-3-cyclohexenecarbaldehyde;
- $C_4$-$C_{18}$ cycloaliphatic ketones, preferably 1-(3,3-dimethylcyclohexyl)-4-penten-1-one, 1-(5,5-dimethyl-1-cyclohexen-1-yl)-4-penten-1-one, 2,3,8,8-tetramethyl-1,2,3,4,5,6,7,8-octahydro-2-naphthalenyl methyl ketone, methyl-2,6,10-trimethyl-2,5,9-cyclododecatrienyl ketone, tert-butyl(2,4-dimethyl-3-cyclohexen-1-yl)ketone;
- esters of cyclic alcohols in $C_4$-$C_{18}$, preferably 2-tert-butylcyclohexyl acetate, 4-tert-butyl-cyclohexyl acetate, 2-tert-pentylcyclohexyl acetate, 4-tert-pentylcyclohexyl acetate, decahydro-2-naphthyl acetate, 3-pentyltetrahydro-2H-pyran-4-yl acetate, decahydro-2,5,5,8a-tetramethyl-2-naphthyl acetate, 4,7-methano-3a,4,5,6,7,7a-hexahydro-5 or 6-indenyl acetate, 4,7-methano-3a,4,5,6,7,7a-hexahydro-5 or 6-indenyl propionate, 4,7-methano-3a,4,5,6,7,7a-hexahydro-5 or 6-indenyl isobutyrate, 4,7-methanooctahydro-5 or 6-indenyl acetate;
- esters of cycloaliphatic carboxylic acids in $C_4$-$C_{18}$, preferably allyl 3-cyclohexylpropionate, allyl cyclohexyloxyacetate, methyl dihydrojasmonate, methyl jasmonate, methyl 2-hexyl-3-oxocyclopentanecarboxylate, ethyl 2-ethyl-6,6-dimethyl-2-cyclohexenecarboxylate, ethyl 2,3,6,6-tetramethyl-2-cyclohexenecarboxylate, ethyl 2-methyl-1,3-dioxolane-2-acetate;
- $C_4$-$C_{18}$ aromatic hydrocarbons, preferably styrene and diphenylmethane;
- $C_4$-$C_{18}$ araliphatic alcohols, preferably benzyl alcohol, 1-phenylethyl alcohol, 2-phenylethyl alcohol, 3-phenylpropanol, 2-phenylpropanol, 2-phenoxyethanol, 2,2-dimethyl-3-phenylpropanol, 2,2-dimethyl-3-(3-methylphenyl)propanol, 1,1-dimethyl-2-phenylethyl alcohol, 1,1-dimethyl-3-phenylpropanol, 1-ethyl-1-methyl-3-phenylpropanol, 2-methyl-5-phenylpentanol, 3-methyl-5-phenylpentanol, 3-phenyl-2-propen-1-ol, 4-methoxybenzyl alcohol, 1-(4-isopropylphenyl)ethanol;
- esters of araliphatic alcohols in $C_4$-$C_{18}$ and aliphatic carboxylic acids in $C_4$-$C_{18}$, preferably benzyl acetate, benzyl propionate, benzyl isobutyrate, benzyl isovalerate, 2-phenylethyl acetate, 2-phenylethyl propionate, 2-phenylethyl isobutyrate, 2-phenylethyl isovalerate, 1-phenylethyl acetate, alpha-trichloromethylbenzyl acetate, alpha,alpha-dimethylphenylethyl acetate, alpha,alpha-dimethylphenylethyl butyrate, cinnamyl acetate, 2-phenoxyethyl isobutyrate, 4-methoxybenzyl acetate;
- $C_2$-$C_{18}$ araliphatic ethers, preferably 2-phenylethyl methyl ether, 2-phenylethyl isoamyl ether, 2-phenylethyl 1-ethoxyethyl ether, phenylacetaldehyde dimethyl acetal, phenylacetaldehyde diethyl acetal, hydratropaldehyde dimethyl acetal, phenylacetaldehyde glycerol acetal, 2,4,6-trimethyl-4-phenyl-1,3-dioxane, 4,4a,5,9b-tetrahydroindeno[l,2-d]-m-dioxin, 4,4a,5,9b-tetrahydro-2,4-dimethylindeno[1,2-d]-m-dioxin;
- $C_4$-$C_{18}$ aromatic and araliphatic aldehydes, preferably benzaldehyde, phenylacetaldehyde, 3-phenylpropanal, hydratropaldehyde, 4-methylbenzaldehyde, 4-methylphenylacetaldehyde, 3-(4-ethylphenyl)-2,2-dimethylpropanal, 2-methyl-3-(4-isopropylphenyl)propanal, 2-methyl-3-(4-tert-butylphenyl)propanal, 3-(4-tert-butylphenyl)propanal, cinnamaldehyde, alpha-butylcinnamaldehyde, alpha-amylcinnamaldehyde, alpha-hexylcinnamaldehyde, 3-methyl-5-phenylpentanal, 4-methoxybenzaldehyde, 4-hydroxy-3-methoxybenzaldehyde, 4-hydroxy-3-ethoxybenzaldehyde, 3,4-methylenedioxybenzaldehyde, 3,4-dimethoxybenzaldehyde, 2-methyl-3-(4-methoxyphenyl)propanal, 2-methyl-3-(4-methylenedioxyphenyl)propanal;
- $C_4$-$C_{18}$ aromatic and araliphatic ketones, preferably acetophenone, 4-methylacetophenone, 4-methoxyacetophenone, 4-tert-butyl-2,6-dimethylacetophenone, 4-phenyl-2-butanone, 4-(4-hydroxyphenyl)-2-butanone, 1-(2-naphthalenyl)ethanone, benzophenone, 1,1,2,3,3,6-hexamethyl-5-indanyl methyl ketone, 6-tert-butyl-1,1-dimethyl-4-indanyl methyl ketone, 1-[2,3-dihydro-1,1,2,6-tetramethyl-3-(1-methylethyl)-1H-5-indenyl]ethanone, 5',6',7',8'-tetrahydro-3',5',5',6',8',8'-hexamethyl-2-acetonaphthone;
- $C_4$-$C_{18}$ aromatic and araliphatic carboxylic acids and esters thereof, preferably phenylacetic acid, methyl benzoate, ethyl benzoate, hexyl benzoate, benzyl benzoate, methyl phenylacetate, ethyl phenylacetate, geranyl phenylacetate, phenylethyl phenylacetate, methyl cinnamate, ethyl cinnamate, benzyl cinnamate, phenylethyl cinnamate, cinnamyl cinnamate, allyl phenoxyacetate, methyl salicylate, isoamyl salicylate, hexyl salicylate, cyclohexyl salicylate, cis-3-hexenyl salicylate, benzyl salicylate, phenylethyl salicylate, methyl 2,4-dihydroxy-3,6-dimethylbenzoate, ethyl 3-phenylglycidate, ethyl 3-methyl-3-phenylglycidate;
- nitrogen-containing aromatic compounds in $C_4$-$C_{18}$, preferably 2,4,6-trinitro-1,3-dimethyl-5-tert-butylbenzene, 3,5-dinitro-2,6-dimethyl-4-tert-butylacetophenone, cinnamonitrile, 5-phenyl-3-methyl-2-pentenenitrile, 5-phenyl-3-methylpentanenitrile, methyl anthranilate, methyl N-methylanthranilate, Schiff bases of methyl anthranilate with 7-hydroxy-3,7-dimethyloctanal, 2-methyl-3-(4-tert-butylphenyl)propanal, 2,4-dimethyl-3-cyclohexene-carbaldehyde, 6-isopropylquinoline, 6-isobutylquinoline, 6-sec-butylquinoline, indole, skatole, 2-methoxy-3-isopropylpyrazine, 2-isobutyl-3-methoxypyrazine;

- phenols, phenyl ethers and phenyl esters, preferably estragole, anethole, eugenol, eugenyl methyl ether, isoeugenol, isoeugenyl methyl ether, thymol, carvacrol, diphenyl ether, beta-naphthyl methyl ether, beta-naphthyl ethyl ether, beta-naphthyl isobutyl ether, 1,4-dimethoxybenzene, eugenyl acetate, 2-methoxy-4-methylphenol, 2-ethoxy-5-(1-propenyl)phenol, p-cresyl phenylacetate;
- heterocyclic compounds in $C_4$-$C_{12}$, preferably 2,5-dimethyl-4-hydroxy-2H-furan-3-one, 2-ethyl-4-hydroxy-5-methyl-2H-furan-3-one, 3-hydroxy-2-methyl-4H-pyran-4-one, 2-ethyl-3-hydroxy-4H-pyra n-4-one;
- lactones in $C_4$-$C_{12}$, preferably 1,4-octanolide, 3-methyl-1,4-octanolide, 1,4-nonanolide, 1,4-decanolide, 8-decen-1,4-olide, 1,4-undecanolide, 1,4-dodecanolide, 1,5-decanolide, 1,5-dodecanolide, 1,15-pentadecanolide, cis and trans-11-pentadecen-1,15-olide, cis- and trans-12-pentadecen-1,15-olide, 1,16-hexadecanolide, 9-hexadecen-1,16-olide, 10-oxa-1,16-hexadecanolide, 11-oxa-1,16-hexadecanolide, 12-oxa-1,16-hexadecanolide, ethylene 1,12-dodecanedioate, ethylene 1,13-tridecanedioate, coumarin, 2,3-dihydrocoumarin, octahydrocoumarin.

In one embodiment, the fragrance composition contains from about 1.0 to about 25.0 wt% of fragrance. The fragrance composition preferably contains from about 1.0 to about 20.0 wt%, more preferably from about 1.0 to about 15.0 wt%, more preferably from about 2.5 to about 15.0 wt%, more preferably from about 5.0 to about 15.0 wt% of fragrance.

Fragrances often include solvents which may be used at levels up to 30.0% by weight of the fragrance. Solvents are defined as relatively low odour liquids which can dissolve a target material in reasonable proportions. For perfumery use solvents may be defined as liquids having sufficiently little odour that they can be added at 30.0% by weight to a fragrance without substantially changing the odour of that fragrance. Solvents are used in the fragrance industry to dilute olfactively powerful ingredients and to facilitate the handling of solid ingredients by dissolving them and handling them as liquids. Examples of suitable solvents include hydrophobic materials, which include hydrocarbons such as those sold under the trade name Isopar®, alkyl esters such as isopropyl myristate, dialkyl adipates, dialkyl succinates, dialkyl glutarates, (such as the dimethyl esters sold under the trade name Flexisolv®), citrate esters (such as acetyl triethyl citrate and acetyl tributyl citrate), diethyl phthalate and benzyl benzoate. Examples of water miscible solvents include diethylene glycol monoethyl ether, 3-methoxy-3-methyl-1-butanol, dipropylene glycol and isopropylidene glycerol sold under the trade name Augeo™ clean multi. By water miscible is meant solvents which are soluble in water at 25°C at more than 30% by weight.

**Diol and surfactant system**

The fragrance composition of the invention comprises from about 2.0 to about 30.0 wt% of a system comprising, such as consisting of, a diol and a surfactant. In one embodiment, the fragrance composition comprises from about 5.0 to about 25.0 wt%, preferably from about 10.0 to about 25.0 wt% of the system.

In one embodiment, the system:fragrance weight ratio is in the range from about 5:1 to about 0.8:1, preferably in the range from about 3.5:1 to about 0.8:1, more preferably in the range from about 3:1 to about 1:1.

In one embodiment, the surfactant:diol weight ratio is in the range from about about 4:1 to about 0.5:1.

*Diols*

Diols useful in the present invention includes vicinal diols, non-vicinal diols and mixtures thereof. In the context of the present invention ether diols, such as diethylene glycol and dipropylene glycol, are not encompassed within the definition of the term "diol". Vicinal diols include those having from 3 to 8 carbon atoms, preferably from 4 to 8 carbon atoms, and more preferably from 5 to 8 carbon atoms, such as 1,2 propylene glycol, 1,2 butanediol, 1,2-pentanediol, 1,2-hexanediol, 1,2-heptanediol, 1,2-octanediol and mixtures thereof. Non-vicinal diols, i.e. diols with the two alcohol groups not on adjacent carbon atoms, include those having from 3 to 12 carbon atoms, preferably from 4 to 8 carbon atoms, such as 1,3-butane diol (or 1,3-butylene glycol), pentylene glycol, 2-methylpentan-2,4-diol (or hexylene glycol), octylene glycol and mixtures thereof.

In one embodiment, the diol is a non-vicinal diol or a mixture of non-vicinal diols.

In another embodiment, the diol:fragrance weight ratio is in the range from about 2:1 to about 0.3:1, preferably in the range from about 1.5:1 to about 0.8:1.

*Surfactants*

As used herein, the term surfactant means an amphiphilic molecule i.e. containing a hydrophilic and a hydrophobic part which is surface active in that it lowers the surface tension of water and can form sub microscopic molecular assemblies at concentrations exceeding a specific concentration (critical micelle concentration) in aqueous solution. The hydrophilic part of the surfactants may have anionic, amphoteric, zwitterionic or nonionic chemical functional groups.

In the context of the present invention the surfactant is not a phospholid. The generic term "phospholipid" is intended to encompass (in a non-exhaustive way) glycerophospholipids, phosphatidic acid, phosphatidylethanolamine, phosphatidylcholine, phosphatidylserine, phosphatidylinositol, phosphatidylinositol phosphate, phosphatidylinositol biphosphate, phosphatidylinositol triphosphate, sphingolipids, ceramide phosphorylcholine, ceramide phosphorylethanolamine.

In one embodiment, the surfactant is selected from anionic surfactants, cationic surfactants, zwitterionic surfactants, amphoteric surfactants, nonionic surfactants and mixtures thereof. Any such surfactants can be used in the fragrance composition of the invention as long as they are cosmetically acceptable, i.e. they are skin mild as determined by a zein solubilisation test with the individual surfactants having zein numbers below 400, preferably below 300 and more preferably below 200. The zein test is described as protocol n°26 on the website of the Joint Research Centre of the European Commission (URL: https://ecvam-dbalm.jrc.ec.europa.eu/methods-and-protocols) and consists in determining the amount of the corn protein, zein, dissolved by a surfactant as mg nitrogen in 100ml of surfactant solution, the value of which is referred to as the "zein number". It should be borne in mind that mixtures of cationic and anionic surfactants are not to be used in the context of the present invention.

*Anionic surfactants*

Anionic surfactants useful in the present invention have at least one hydrophobic chain containing between 4 and 20 carbon atoms. Preferred surfactants may contain multiple hydrophobic chains, i.e. at least 2 separate linear or branched alkyl, or alkaryl (i.e. an alkyl chain with an aromatic substituent such as a benzyl group) moieties as shown in the formula below:

$$R-X^--R' \, M^+$$

wherein:

X represents any of the common polar hydrophilic groups known in anionic surfactants for example carboxylate, sulphate, sulphonate, ether sulphate, sulphosuccinate, glutamate, or phosphate;
R represents a linear or branched alkyl group, or an alkaryl group each having from 4 to 20 carbon atoms, preferably from 4 to 16 carbon atoms and more preferably from 4 to 12 carbon atoms;
R' can be either a hydrogen atom or R; and
$M^+$ is a cosmetically acceptable cation as defined below.

In one embodiment, the anionic surfactant is selected from the group consisting of:

- an alkyl benzene sulfonate having the formula $R-Ar-SO_3^-M^+$ wherein R is a $C_4$-$C_{20}$ alkyl or alkenyl group, Ar is a phenyl group, and $M^+$ is a cosmetically acceptable cation, for example selected from the group consisting of ammonium ion, a charged alkanolamine ion (such as a cationic triethanolamine), an alkali metal ion (such as sodium or potassium ions), and mixtures thereof;
- an alkyl sulfate having the formula $R-OSO_3^-M^+$, wherein R and $M^+$ are each defined as above;
- an alkyl ether sulfate having the formula $R-O(C_2H_4O)_ySO_3^-M^+$, wherein R and $M^+$ are each independently as defined above and y is an integer comprised between 1 and 10;
- an alkyl phosphate having the formula $RO-P(=O)(OR')O^-M^+$, wherein R, R' and $M^+$ are each independently as defined above. Preferably the alkyl phosphate is a dialkyl phosphate;
- an alkyl isethionate having the formula $R-O-C(=O)-C_2H_4-SO_3^-M^+$, wherein R and $M^+$ are each defined as above;
- an alkyl glutamate having the formula $R-(C=O)-NH-(C_2H_4COOH)-COO^-M^+$, wherein R and $M^+$ are each defined as above;
- a sulfosuccinate, and preferably a dialkyl sulfosuccinate; examples of sulfosuccinates include disodium N-octadecylsulfosuccinate, disodium lauryl sulfosuccinate, diammonium lauryl sulfosuccinate, tetrasodium N-(1,2-dicarboxyethyl)-N-octadecylsulfosuccinate, diamyl ester of sodium sulfosuccinic acid, dihexyl ester of sodium sulfosuccinic acid, dioctyl esters of sodium sulfosuccinic acid, and mixtures thereof.

In one embodiment, the anionic surfactant is selected from dialkyl glutamates, dialkyl phosphates, dialkyl sulfosuccinates and mixtures thereof. In one embodiment the anionic surfactant is a dialkyl sulfosuccinate such as those sold under the Aerosol™ name by Cytec Industries, or those sold under the Tego™ name by Glenn Corporation, e.g.

sodium diethylhexyl sulfosuccinate sold as Aerosol™ OT or as Tego™ sulfosuccinate DO 75.

In another embodiment, the anionic surfactant is selected from: ammonium lauryl sulfate, ammonium laureth sulfate, triethylamine lauryl sulfate, triethylamine laureth sulfate, triethanolamine lauryl sulfate, triethanolamine laureth sulfate, monoethanolamine lauryl sulfate, monoethanolamine laureth sulfate, diethanolamine lauryl sulfate, diethanolamine laureth sulfate, lauric monoglyceride sodium sulfate, sodium lauryl sulfate, sodium laureth sulfate, potassium lauryl sulfate, potassium laureth sulfate, ammonium cocoyl sulfate, ammonium lauroyl sulfate, sodium cocoyl sulfate, sodium lauroyl sulfate, potassium cocoyl sulfate, potassium lauryl sulfate, triethanolamine lauryl sulfate, triethanolamine lauryl sulfate, monoethanolamine cocoyl sulfate, monoethanolamine lauryl sulfate, sodium tridecyl benzene sulfonate, sodium dodecyl benzene sulfonate, sodium diethylhexyl sulfosuccinate, sodium dioctyl sulfosuccinate and mixtures thereof.

*Cationic surfactants*

Cationic surfactants useful in the present invention contain amino or quaternary ammonium hydrophilic moieties which are positively charged when dissolved in the composition of the present invention.

In one embodiment the cationic surfactant corresponds to the general formula:

$$[N(R_1)(R_2)(R_3)(R_4)]^+(X)$$

wherein $R_1$, $R_2$, $R_3$, and $R_4$ are independently selected from (a) an aliphatic group of from 1 to 22 carbon atoms, or (b) an aromatic, alkoxy, polyoxyalkylene, alkylamido, hydroxyalkyl, aryl or alkylaryl group having up to 22 carbon atoms. The quaternary nitrogen compound can also be a component of a heterocyclic nitrogen-containing moiety, such as morpholine or pyridine. X is a salt-forming anion such as halogen, (e.g. chloride, bromide), acetate, citrate, lactate, glycolate, phosphate nitrate, sulphate, methosulphate or ethosulphate. The aliphatic groups can contain, in addition to carbon and hydrogen atoms, ether linkages and or ester linkages, and other groups such as amino groups. The longer chain aliphatic groups, e.g., those of about 12 carbons, or higher, can be saturated or unsaturated and are preferably derived from natural sources of fatty acids such as coconut oil, palm oil, palm kernel oil, or tallow.

In another embodiment, the cationic surfactant is a monoalkyl quaternary ammonium compound in which one alkyl chain length is $C_{10}$ to $C_{24}$. Examples of such surfactants are selected from: cetyltrimethylammonium chloride, behenyltrimethylammonium chloride, cetylpyridinium chloride, dodecyltrimethylammonium chloride, hexadecyltrimethylammonium chloride, octyldimethylbenzylammonium chloride, decyldimethylbenzylammonium chloride, stearyldimethylbenzylammonium chloride, didodecyldimethylammonium chloride, dioctadecyldimethylammonium chloride, tallowtrimethylammonium chloride, cocotrimethylammonium chloride, PEG-2 oleylammonium chloride, salts of these where the chloride is replaced by halogen, (e.g. bromide), acetate, citrate, lactate, glycolate, phosphate nitrate, sulphate, or alkylsulphate, and mixtures thereof.

In another embodiment, the cationic surfactant is a salt of a primary, secondary, or tertiary fatty amine. The alkyl groups of such amines preferably have from about 10 to about 24 carbon atoms, and can be substituted or unsubstituted. Particularly useful are amido substituted tertiary fatty amines. Examples of the latter amines are selected from: stearamidopropyldimethylamine, stearamidopropyldiethylamine, stearamido-ethyldiethylamine, stearamidoethyldimethylamine, palmitamidopropyldimethylamine, palmitamidopropyldiethylamine, palmitamidoethyldiethylamine, palmitamido-ethyldimethylamine, behenamidopropyldimethylamine, behenamidopropyldiethylamine, behenamidoethyldiethylamine, behenamidoethyldimethylamine, arachidamido-propyldimethylamine, arachidamidopropyldiethylamine, arachidamidoethyldiethylamine, arachidamidoethyldimethylamine, diethylaminoethylstearamide, and mixtures thereof.

In another embodiment, the cationic surfactant is an amine used in combination with an acid to provide the cationic species. Suitable amines are selected from: dimethylstearamine, dimethylsoyamine, soyamine, myristylamine, tridecylamine, ethylstearylamine, N-tallowpropanediamine, ethoxylated (with 5 moles of ethylene oxide) stearylamine, dihydroxyethylstearylamine, and arachidylbehenylamine. Suitable acids are selected from: L-glutamic acid, L-glutamic acid hydrochloride, lactic acid, hydrochloric acid, malic acid, succinic acid, acetic acid, fumaric acid, tartaric acid, and citric acid, preferably from: L-glutamic acid, lactic acid, and citric acid.

The molar ratio of protonatable amines to H from the acid is preferably from about 1:0.3 to 1:1.2, and more preferably from about 1:0.5 to about 1:1.1.

In the compositions of the invention, the level of cationic surfactant - when used - is preferably from about 0.1 to about 20, more preferably from about 0.5 to about 15, most preferably from about 0.5 to about 10% by weight of the total composition.

*Amphoteric and zwitterionic surfactants*

Amphoteric or zwitterionic surfactants useful in the present invention include those which are known for use in personal care and cosmetics. Non limiting examples of suitable zwitterionic or amphoteric surfactants are described in US patents 5,104,646 and 5,106,609.

In one embodiment, amphoteric surfactants include those surfactants broadly described as derivatives of aliphatic secondary and tertiary amines in which the aliphatic radical can be straight or branched chain and wherein one of the aliphatic substituents contains from about 8 to about 20 carbon atoms and one contains an anionic group such as carboxy, sulfonate, sulfate, phosphate, or phosphonate. In another embodiment, the amphoteric surfactants are selected from: cocoamphoacetate, cocoamphodiacetate, lauroamphoacetate, lauroamphodiacetate, and mixtures thereof.

In one embodiment, zwitterionic surfactants include those surfactants broadly described as derivatives of aliphatic quaternary ammonium, phosphonium, and sulfonium compounds, in which the aliphatic radicals can be straight or branched chain, and wherein one of the aliphatic substituents contains from about 8 to about 20 carbon atoms and one contains an anionic group such as carboxy, sulfonate, sulfate, phosphate or phosphonate. In another embodiment, zwitterionics such as betaines are selected.

*Non-ionic surfactants*

Non-ionic surfactants useful in the present invention include ethoxylated and/or propoxylated $C_4$-$C_{20}$ aliphatic alkyl ethers containing 5 to 60 EO or PO units. The aliphatic groups can be petrochemically derived but are preferably derived from vegetable oils such as coconut oil, palm oil, rapeseed oil etc. and include both saturated and unsaturated alkyl chains. Commercial examples of suitable alkyl ethoxylate ethers are those known with the trade name Genapol™. Other suitable non-ionic surfactants are the polyethylene glycol sorbitan esters containing 3 to 30 ethoxylate units such as sorbitan esters with lauric, oleic, myristic, stearic, and palmitic acids known under the tradenames Tween™ from Croda or Glycosperse™ from Lonza. Other suitable non-ionic surfactants are the ethoxylated polyglyceryl esters of $C_8$-$C_{20}$ fatty acids with 20-80 ethoxylate groups, such as glycerol-polyethylene glycol oxystearate commercialized by BASF under the trade name Cremophor™. Polyglyceryl esters are also suitable non-ionic surfactants, these esters comprise from 1 to 10 glycerin units and one or two $C_4$ to $C_{20}$ aliphatic alkyl groups. Examples are available under the Polyaldo™ trade name from Lonza.

Further suitable non-ionic surfactants are the N-alkyl-N-acyl glucamines (also referred to as glucamides) where the acyl groups are derived from $C_8$-$C_{20}$ fatty acids. Preferably the fatty acids are derived from plant materials such as coconut oil and palm oil. Examples of these surfactants include lauroyl/myristoyl methyl glucamide, Cocoyl methyl glucamide and capryloyl/caproyl methyl glucamide. Commercially these surfactants are available as Glucotain Flex™ , Glucotain Care™ and Glucotain Clear™ respectively.

Other suitable non-ionic surfactants are the $C_6$-$C_{20}$ ethers or esters of mono-, di- or polysaccharides. Suitable non-ionic surfactants are the sucrose esters with $C_8$-$C_{20}$ fatty acid, such as sucrose esters with lauric, oleic, palmitic or stearic acid, such as the Ryoto™ Sugar Esters commercialized by Mitsubishi-Kagaku Foods Corporation. Other saccharide surfactants include the Oramix™ range by Seppic, the Sepiclear™ range by Seppic such as Sepiclear™ G7 or the Sisterna™ range such as L70-C by Sisterna. Further suitable non-ionic surfactants are ($C_6$-$C_{16}$)alkyl glucosides, such as those sold under the Plantacare™ name by Cognis, e.g. $C_{12}$-$C_{16}$ fatty alcohol polyglycoside sold as Plantacare™1200UP.

In one embodiment, the non-ionic surfactant is selected from: sorbitan esters with lauric, oleic, myristic, stearic, or palmitic acid; sucrose esters with $C_8$-$C_{20}$ fatty acid; polyglyceryl esters comprising from 1 to 10 glycerin units and one or two $C_4$ to $C_{20}$ aliphatic alkyl groups; ($C_6$-$C_{16}$) alkyl glucosides; glucamides; and mixtures thereof.

Further examples of anionic, cationic, zwitterionic, amphoteric and non-ionic surfactants useful in the present invention are described in McCutcheon's Surfactants and Detergents, North American & International Editions from MC publishing Glen Rock, New Jersey USA which is published annually. The surfactants used are preferably those approved for use in cosmetic and personal care products i.e. they are approved for use under the regulations of the European Union Cosmetics Directive (76/768/EEC) and may be found in Annex II listed under the functions of emulsifying agents, cleansing agents, surfactants, or hydrotropes.

In one embodiment, the surfactant is selected from anionic surfactants, non-ionic surfactants and mixtures thereof. In one embodiment the surfactant is an anionic surfactant or a mixture of anionic surfactants as defined above. In one embodiment, the surfactant is a non-ionic surfactant or a mixture of non-ionic surfactants as defined above. In one embodiment, the surfactant is a mixture of one or more anionic surfactant(s) and one or more non-ionic surfactant(s) as defined above. The weight ratio of non-ionic surfactant(s) to anionic surfactant(s) is preferably in the range of from about 3:1 to about 1:3. In a preferred embodiment, the weight ratio of non-ionic surfactant(s) to anionic surfactant(s) is in the range of from about 2:1 to about 1:1.5.

**Ethanol**

The fragrance composition of the invention comprises from about 25.0 to about 80.0 wt%, preferably from about 30.0 to about 80.0 wt%, more preferably from about 35.0 to about 80.0 wt%, more preferably from about 35.0 to about 70.0 wt% ethanol, more preferably from about 35.0 to about 60.0 wt%, more preferably from about 35.0 to about 50.0 wt%, of ethanol.

**Water**

The fragrance composition of the invention also comprises at least about 5.0 wt%, preferably at least about 10.0 wt%, preferably at least about 15.0 wt%, more preferably at least about 20.0 wt%, of water. The amount of water is such that water and ethanol together make up at most 97.0 wt% of the composition.

**Additional Ingredients**

Other ingredients that may optionally be present in the fragrance compositions of the invention include for example antioxidants, chelating agents, UV filters, and chemaesthetic agents such as cooling agents. The amount of optional ingredients will vary depending on the purpose and effectiveness of the ingredient. Typically, such ingredients represent from about 0.0005% to about 2.5% by weight, preferably from about 0.001% to about 1% by weight, more preferably from about 0.01% to about 0.5% by weight, of the composition.

**Preparation**

The fragrance compositions of the present invention may be prepared, for example, by mixing all of the ingredients, for example by hand stirring or if needed by using a mechanical mixer such as a stirrer to form a clear homogeneous mixture.

In one embodiment, where a mixture of non-ionic and anionic surfactants is used, the non-ionic surfactant is added to the fragrance and any oil soluble optional ingredients, and the mixture is stirred. Then the diol or mixture of diols is added with stirring. Separately the anionic surfactant is dissolved or dispersed in water along with any water-soluble optional ingredients with warming if necessary. The aqueous solution or dispersion is then added slowly to the organic phase with constant gentle stirring. After the aqueous phase has been added the appropriate amount of ethanol is added, it may then be necessary to add a further small amount of diol to ensure absolute clarity.

**Uses**

The fragrance composition of the invention is transparent and can accordingly be advantageously used as a fine fragrance composition, preferably in the form of a perfume concentrate, a perfume, an eau de toilette (EdT), an eau de parfum, a cologne or a body mist.

In one embodiment, the fragrance composition of the invention is in the form of a spray.

Preferably, the composition is allowed to dry after its application on the substrate and the substrate is preferably the skin.

The invention is illustrated by but not limited to the examples below.

**Example 1**

A fragrance (hereafter fragrance TAK, the formulation of which is shown in table 1) was formulated in a typical Eau de Toilette (EdT1) or in fragrance compositions according to the invention (compositions C1 to C4), as shown in table 2. The amount of individual fragrance ingredients retained on paper substrate (blotter) after set time periods was assessed by the following method.

*Test Method*

100μL samples of either EdT1 or compositions C1 to C4 were applied to 4.5cm long standard paper blotters with a microsyringe. Three replicate blotters were prepared for each extraction time. After the appropriate evaporation time each of the 3 blotters was transferred to a 30mL glass bottle. 3mL of ethanol and 50μL of a 1% solution of methyl decanoate in ethanol as an internal standard were added. The bottle was sealed and placed on a roller bed to extract the blotter for 1 hour. A sample of the extracting solution was transferred to a vial which was sealed and analysed by gas chromatography (GC 6890) with a Mass Spectrometer detector (MS 5973) using the method described below. The results of the three replicate measurements were averaged. At exact times the same extraction was repeated on the remaining blotters which were stored on a warm surface at 32°C open to the air to allow evaporation. The gas chromatography conditions were as given below.

**Oven conditions**

Initial Temp: 50°C
Initial Time: 2 min
Rate: 10°C /min
Final Temp: 280°C
Final Time: 5 min
Run Time: 30 min

**Column**

Column: HP-5MS 30m * 0.25mm * 0.25$\mu$m
Gas: Helium
Constant Pressure 18.55 psi

**Inlet**

Split mode Ratio 40:1
Inlet Temp: 250°C

**Detector**

MS 5973
Mass range: 35 to 350
Threshold: 100
Mass Quad: 150°C
Mass Source: 230°C

To simplify the results the average amount of each fragrance ingredient from the 3 samples measured at time zero was set at 100% and all other measurements were then related to that value so tables 3 to 6 show decreasing amounts of fragrance ingredients as a percentage of the initial amount.

Table 1

| Ingredients | | CAS No | ClogP | Category | % |
|---|---|---|---|---|---|
| Hexyl Acetate | A | 142-92-7 | 2.83 | Top | 11.11 |
| Limonene | B | 5989-27-5 | 4.35 | Top | 11.11 |
| Linalool | C | 78-70-6 | 2.75 | Top | 11.11 |
| Linalyl acetate | D | 115-95-7 | 3.70 | Heart | 11.11 |
| Geraniol | E | 106-24-1 | 2.97 | Heart | 11.11 |
| Verdox™ | F | 88-41-5 | 4.06 | Heart | 11.11 |
| Lilial™ | G | 80-54-6 | 4.10 | Heart | 11.11 |
| Cis-3-hexenyl salicylate | H | 65405-77-8 | 4.50 | Bottom | 11.11 |
| Ethylene Brassylate | I | 105-95-3 | 3.02 | Bottom | 11.11 |

Each fragrance ingredient was classified into one of 3 groups (top note/heart note/bottom note) according to its volatility as measured with the retention index on a GC equipped with a non polar column. The retention indexes were determined as below:

*Retention Index definition*

The elution order of fragrance ingredients on a GC equipped with a non-polar column is closely linked to the volatility of ingredients: the more volatile an ingredient is, the shorter the elution (or retention) time is.

Since the elution time is linked to the temperature program of the GC oven, it may change depending on the GC oven program. However the Retention Index parameter (which is a function of Retention Time) is not dependent on the GC oven program: for a given column the Retention Index is indeed unique for an ingredient whatever the GC oven program is. Therefore the Retention Index of fragrance ingredients can be used to classify said fragrance ingredients according to their volatility. Information about the Retention Index can be found in "Fragrance Chemistry - The Science of the Sense of Smell edited by E T Theimer Published by Academic Press in 1982 ( ISBN 0-12-685850-0)", and references therein.

*Retention Index calculation*

The Retention Index was calculated using a GC equipped with a HP-5MS column using the formula below:

$$RI = 100 \times \left[ n + \frac{rt(unknown) - rt(n)}{rt(n+i) - rt(n)} \right]$$

wherein:

RI = Retention Index
n = carbon number of fatty acid portion of reference ester used
i = carbon number of increment between subsequent reference esters
rt = retention (or elution) time

The Retention index of the following ethyl esters was determined to be:

Ethyl Butyrate (n=4)     RI = 400
Ethyl Pentanoate (n=5)   RI = 500
Ethyl Caproate (n=6)     RI = 600

The fragrance ingredients were then classified as top note, heart note or bottom note according to their Retention Index:

Top note       RI ≤ 810
Heart note     810 < RI ≤ 1200
Bottom note    RI > 1200

Table 2

| Ingredients | EdT1 | C1 | C2 | C3 | C4 |
|---|---|---|---|---|---|
| Fragrance TAK | 10.0 | 10.00 | 10.00 | 10.0 | 10.0 |
| Aerosol™ OT 100 | | 5.00 | 5.00 | 5.00 | 5.00 |
| Propylene glycol | | 2.00 | 2.00 | 2.00 | 2.00 |
| Hexylene Glycol | | 8.00 | 8.00 | 8.00 | 8.00 |
| Tween ™ 20 | | 9.88 | 9.88 | | |
| Dermofeel™ G10L | | | | 9.88 | |
| Sisterna™ L70C | | | | | 9.88 |

(continued)

| Ingredients | EdT1 | C1 | C2 | C3 | C4 |
|---|---|---|---|---|---|
| Ethanol | 65.00 | 45.12 | 25.12 | 45.12 | 45.12 |
| Water | 25.00 | 20.00 | 40.00 | 20.00 | 20.00 |

Table 3

| | T0 | 15 min | | 60 min | | 120 min | | 240 min | | 480 min | |
|---|---|---|---|---|---|---|---|---|---|---|---|
| Ingredients | | EdT1 | C1 | EdT1 | C1 | EdT1 | C1 | EdT1 | C1 | EdT1 | C1 |
| Hexyl acetate | 100 | 13 | 41 | 2 | 5 | 2 | 2 | 1 | 2 | <1 | 2 |
| Limonene | 100 | 1 | 18 | <1 | 1 | <1 | 1 | <1 | 1 | <1 | <1 |
| Linalool | 100 | 57 | 80 | 9 | 61 | 1 | 35 | 1 | 13 | <1 | 3 |
| Linalyl acetate | 100 | 70 | 79 | 27 | 62 | 6 | 39 | 2 | 19 | <1 | 7 |
| Verdox ™ | 100 | 70 | 80 | 29 | 62 | 2 | 39 | <1 | 18 | 1 | 2 |
| Geraniol | 100 | 81 | 82 | 53 | 79 | 28 | 65 | 14 | 52 | 3 | 32 |
| Lilial ™ | 100 | 91 | 78 | 84 | 79 | 75 | 75 | 65 | 72 | 23 | 51 |
| Cis 3-hexenyl salicylate | 100 | 96 | 76 | 87 | 77 | 85 | 75 | 80 | 73 | 32 | 60 |
| Ethylene Brassylate | 100 | 88 | 81 | 85 | 79 | 90 | 81 | 98 | 86 | 100 | 79 |

Table 4

| | T0 | 15 min | | 480 min | |
|---|---|---|---|---|---|
| Ingredients | | EdT1 | C2 | EdT1 | C2 |
| Hexyl acetate | 100 | 15 | 54 | 6 | <1 |
| Limonene | 100 | 2 | 29 | <1 | <1 |
| Linalool | 100 | 58 | 92 | 3 | 4 |
| Linalyl acetate | 100 | 72 | 92 | 2 | 9 |
| Verdox ™ | 100 | 74 | 92 | <1 | 3 |
| Geraniol | 100 | 84 | 95 | 9 | 35 |
| Lilial ™ | 100 | 87 | 98 | 18 | 49 |
| Cis 3-hexenyl salicylate | 100 | 84 | 99 | 32 | 63 |
| Ethylene Brassylate | 100 | 87 | 97 | 73 | 77 |

Table 5

| | T0 | 15 min | | 60 min | | 120 min | | 240 min | | 480 min | | 1440 min | |
|---|---|---|---|---|---|---|---|---|---|---|---|---|---|
| Ingredients | | EdT1 | C3 | EdT1 | C3 | EdT1 | C3 | EdT1 | C3 | EdT1 | C3 | EdT1 | C3 |
| Hexyl acetate | 100 | 13 | 43 | 2 | 5 | 2 | 2 | 1 | 2 | <1 | 2 | <1 | 2 |
| Limonene | 100 | 1 | 19 | <1 | <1 | <1 | <1 | <1 | <1 | <1 | <1 | <1 | <1 |
| Linalool | 100 | 57 | 85 | 9 | 61 | 1 | 37 | <1 | 18 | <1 | 3 | <1 | 1 |
| Linalyl acetate | 100 | 70 | 83 | 26 | 62 | 6 | 41 | 2 | 24 | <1 | 8 | <1 | 3 |
| Verdox ™ | 100 | 70 | 83 | 27 | 64 | 2 | 44 | <1 | 27 | 1 | 3 | <1 | <1 |

(continued)

| Ingredients | T0 | 15 min | | 60 min | | 120 min | | 240 min | | 480 min | | 1440 min | |
|---|---|---|---|---|---|---|---|---|---|---|---|---|---|
| | | EdT1 | C3 | EdT1 | C3 | EdT1 | C3 | EdT1 | C3 | EdT1 | C3 | EdT1 | C3 |
| Geraniol | 100 | 81 | 88 | 53 | 82 | 28 | 73 | 14 | 62 | 3 | 37 | 2 | 15 |
| Lilial ™ | 100 | 91 | 88 | 84 | 92 | 75 | 90 | 65 | 87 | 23 | 60 | 6 | 45 |
| Cis 3-hexenyl salicylate | 100 | 96 | 78 | 87 | 84 | 85 | 83 | 80 | 82 | 32 | 61 | 14 | 50 |
| Ethylene Brassylate | 100 | 88 | 80 | 85 | 93 | 90 | 93 | 98 | 100 | 100 | 82 | 85 | 96 |

Table 6

| Ingredients | T0 | 15 min | | 60 min | | 120 min | | 240 min | | 480 min | |
|---|---|---|---|---|---|---|---|---|---|---|---|
| | | EdT1 | C4 | EdT1 | C4 | EdT1 | C4 | EdT1 | C4 | EdT1 | C4 |
| Hexyl acetate | 100 | 13 | 41 | 2 | 5 | 2 | 5 | 1 | 4 | <1 | 3 |
| Limonene | 100 | 1 | 17 | <1 | <1 | <1 | 1 | <1 | 1 | <1 | 1 |
| Linalool | 100 | 57 | 81 | 9 | 61 | 1 | 28 | <1 | 13 | <1 | 8 |
| Linalyl acetate | 100 | 70 | 82 | 27 | 62 | 6 | 34 | 2 | 18 | <1 | 10 |
| Verdox ™ | 100 | 70 | 83 | 29 | 64 | 2 | 36 | <1 | 18 | 1 | 3 |
| Geraniol | 100 | 81 | 92 | 53 | 82 | 28 | 70 | 14 | 58 | 3 | 42 |
| Lilial ™ | 100 | 91 | 99 | 84 | 92 | 75 | 88 | 65 | 87 | 23 | 52 |
| Cis 3-hexenyl salicylate | 100 | 96 | 92 | 87 | 84 | 85 | 93 | 80 | 94 | 32 | 65 |
| Ethylene Brassylate | 100 | 88 | 100 | 85 | 93 | 90 | 96 | 98 | 100 | 100 | 100 |

The data in tables 3 to 6 show that the fragrance compositions of the invention retain more fragrance on the blotter than a conventional aqueous alcohol EdT. Also the results show a time dependent effect related to the category of the perfume ingredients. Thus, for top note ingredients such as limonene, linalool and hexyl acetate the effect is already noticeable after 15 minutes of evaporation. For heart note ingredients such as lilial or geraniol, the effect is noticeable after at least 60 min of evaporation depending on the surfactant(s) used and the benefit is still present up to 8 hours. Regarding bottom note ingredients such as cis-3-hexenyl salicylate and ethylene brassylate, the amount retained on the blotters is comparable or slightly higher than for a conventional EdT 8 hours after evaporation, and is substantially higher 24 hours after evaporation (see table 5).

**Example 2**

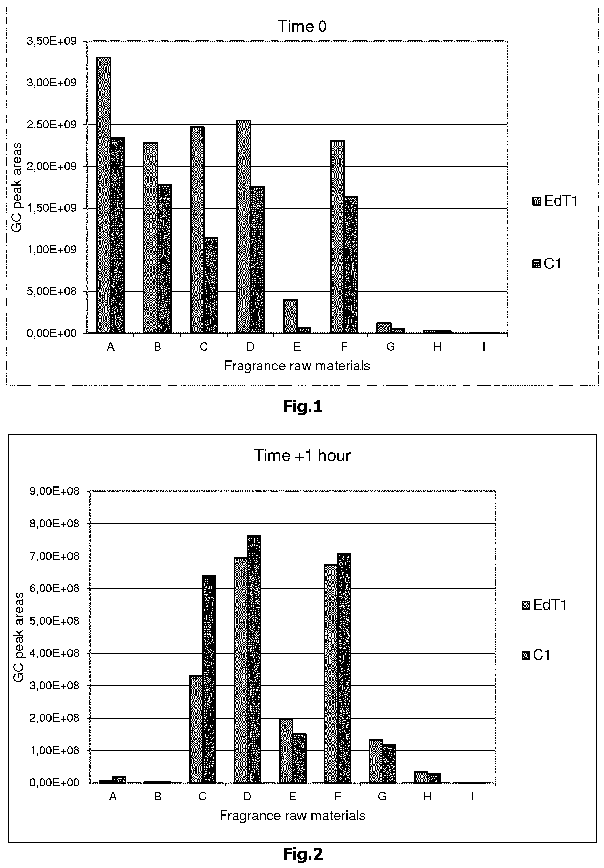

The headspace concentration of individual ingredients of fragrance TAK, as formulated in either a conventional EdT (EdT1) or a fragrance composition of the invention (C1), was assessed by the method described below. The results are shown in figures 1 to 4 respectively. The fragrance ingredients are labeled by letters as indicated in table 1.

*Headspace Test method*

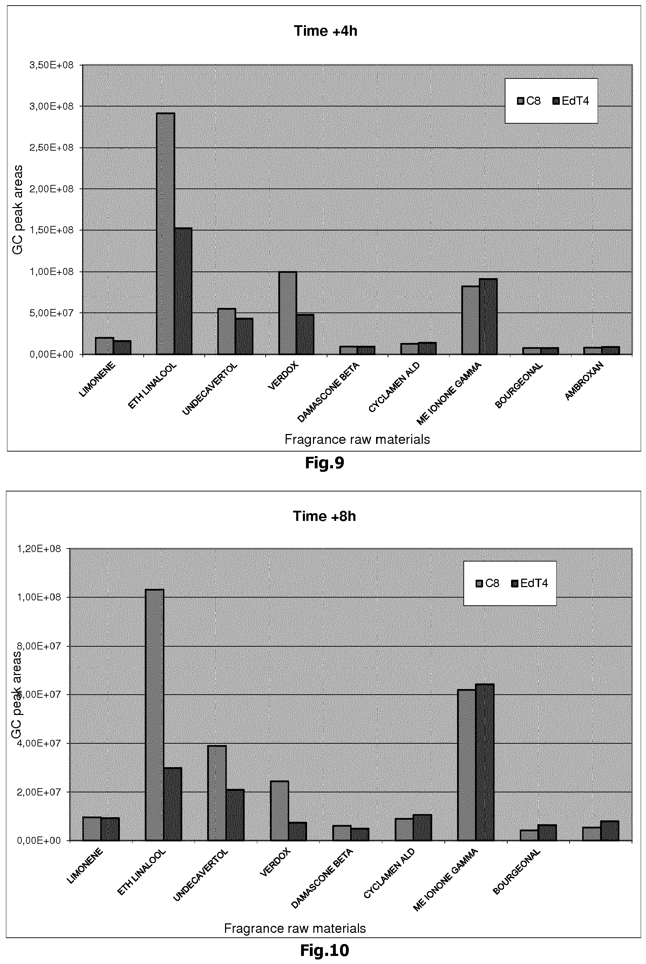

A 10 cm length was cut from a standard perfumers' blotter. A sample of 100μL volume was applied to the upper surface of the blotter with a syringe. The blotter was placed on a hot plate thermostatted at 32°C and left to equilibrate for 5 minutes. Two desorption tubes (6cm length 4mm internal diameter) each containing 2 cm length of Tenax TA adsorbent were fitted to the glass part of a headspace apparatus shown in figure 9. The glass part was fitted over the blotter, a metering pump was connected to one of the desorption tubes and air was drawn through the apparatus flushing the headspace over the blotter at a rate of 17mL/min. Sampling continued for about 16.5 minutes until 280mL of air had been extracted. Then the outlet desorption tube was analysed by gas chromatography as described below. The glass part was removed from the blotter which was left on the hotplate until the next sampling time when two fresh desorption tubes were fitted and the extraction procedure repeated for a further 280mL of extracted headspace.

**Thermal Desorption Gas Chromatography**

GC 7890A
MS 5975C

**Oven conditions**

Initial Temp: 50°C
Initial Time: 5 min
Rate: 3°C /min
Final Temp: 250°C
Final Time: 20 min
Run Time: 91.67 min

**Column:**

Column: HP-5MS 30m * 0.25mm * 0.25μm
Gas: Helium
Constant pressure 12.675 psi

**Inlet**

*Gerstel CIS*

Initial Temp: -30°C
Equilibration Time: 0.05 min
Initial Time: 0.00 min
Rate 12°C / min
End Temp: 260°C
Hold Time: 10 min

*Gerstel TDU*

Temperature program

Initial Temp: 35°C
Delay Time: 0.30 min
Initial Time: 0.00 min
Rate: 30°C / min
End Temp: 250°C
Hold Time: 5.00 min

TDU Settings

Transfer Temp: 250°C
Transfer Temp Mode: Fixed
Desorption Mode: splitless
Sample mode: Retain tube - standby cooling
Standby Temp: 50°C

Detector MS

MS 5973
Mass range: 35 to 350
Threshold: 150
Mass Quad: 150°C
Mass Source: 230°C

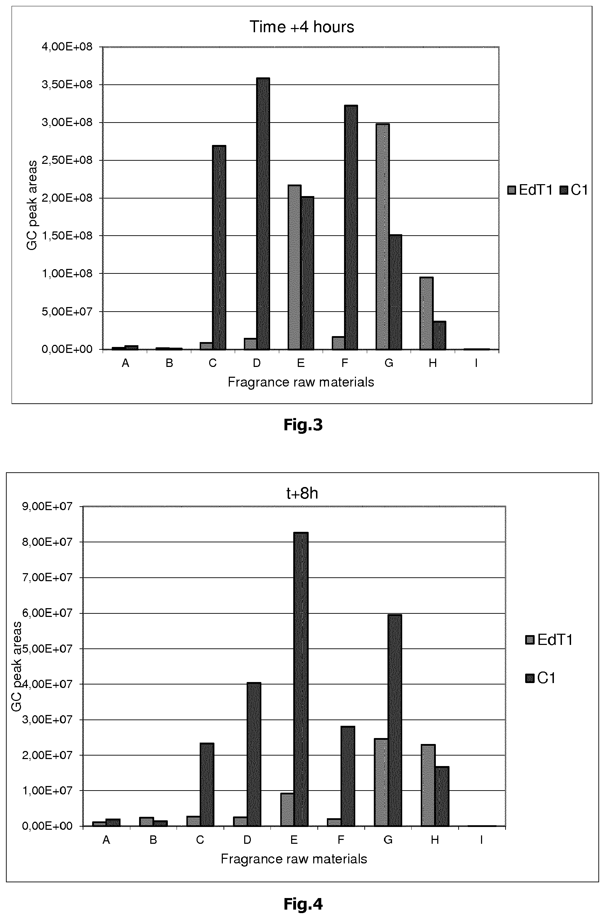

Figure 1 shows that the headspace concentration of the control EdT1 is somewhat higher than that of composition C1 at T0 (for all fragrance ingredients), however this trend is reversed as time progresses. Thus Figure 2 shows that whilst top notes ingredients A and B have been lost after 1 hour for both the control EdT1 and composition C1, a greater headspace concentration was measured for top note ingredient linalool and for heart note ingredients linalyl acetate and Verdox™ in composition C1 (compared to control EdT1), the difference being significant for linalool. Figures 3 and 4 confirm that the headspace concentration is significantly greater for the above-mentioned top and heart note ingredients in composition C1 (compared to control EdT1) at T0 + 4h and T0 + 8h. Figure 4 further shows that the headspace concentration is still much greater in composition C1 for linalool, linallyl acetate and Verdox™. It is also significantly greater for heart note ingredients geraniol and lilial after 8 hours, and is lower for bottom note ingredient cis-3-hexenyl salicylate (compared to control EdT1).

## Example 3

The method of example 1 was repeated using a commercial fragrance (hereafter fragrance COM1, the formulation of which is given in table 7) formulated in a typical Eau de Toilette (EdT2) or in fragrance compositions according to the invention (compositions C5 and C6), as shown in table 8. The results are shown in table 9.

Table 7

| Ingredients | CAS N° | wt% | Category |
|---|---|---|---|
| Grapefruit oil | 8016-20-4 | 9.16 | Top |
| Methyl pamplemousse | 67674-46-8 | 7.30 | Top |
| Methyl heptine carbonate | 111-12-6 | 0.14 | Top |
| Limonene* | 5989-27-5 | 0.23 | Top |
| Ethyl linalool | 10339-55-6 | 9.16 | Top |
| Bourgeonal™ | 18127-01-0 | 0.86 | Heart |
| Citral | 5392-40-5 | 0.86 | Heart |
| Damascone beta, 10 % in TEC** | 35044-68-9 | 2.72 | Heart |
| Citronellol | 106-22-9 | 0.86 | Heart |
| Magnolan™ | 27606-09-3 | 3.58 | Heart |
| Undecavertol™ | 81782-77-6 | 1.86 | Heart |
| Verdox™ | 88-41-5 | 1.86 | Heart |
| Methyl ionone gamma | 127-51-5 | 2.05 | Heart |
| Cyclamen aldehyde | 103-95-7 | 0.54 | Heart |
| Amberketal™, 1% in TEC** | 57345-19-4 | 9.16 | Bottom |
| Habanolide™ | 111879-80-2 | 9.16 | Bottom |
| Hedione™ | 24851-98-7/128087-96-7 | 20.04 | Bottom |
| Muscenone™ | 82356-51-2 | 0.42 | Bottom |
| Orbitone™ | 54464-57-2/166090-45-5 | 18.18 | Bottom |

(continued)

| Ingredients | CAS N° | wt% | Category |
|---|---|---|---|
| Ambroxan™ | 6790-58-5 | 1.86 | Bottom |

*from citrus oil; ** triethyl citrate Bourgeonal™ is 3-(4-tert-butylphenyl)propanal; Magnolan™ is 2,4-dimethyl-4,4a,5,9b-tetrahydroindeno[1,2-d][1,3] dioxine indene; Undecavertol™ is (E)-4-methyldec-3-en-5-ol; Verdoxl™ is (2-tert-butylcyclohexyl) acetate; Amberketal™ is (3aR,5aS,9aS,9bR)-3a,6,6,9a-tetramethyl-2,4,5,5a,7,8,9,9b-octahydro-1H-benzo[e][1]benzofurane; Habanolide™ is (12E)-1-oxacyclohexadec-12-en-2-one; Hedione™ is methyl 3-oxo-2-pentylcyclopentaneacetate;

Muscenone™ is (5E)-3-methylcyclopentadec-5-en-1-one; Orbitone™ is Ethanone, 1-(1,2,3,4,5,6,7,8-octahydro-2,3,8,8-tetramethyl-2-naphthalenyl); Ambroxan™ is (3aR,5aS,9aS,9bR)-3a,6,6,9a-tetramethyl-2,4,5,5a,7,8,9,9b-octahydro-1H-benzo[e][1]benzofuran

Table 8

| Ingredients | EdT2 | C5 | C6 |
|---|---|---|---|
| Fragrance COM1 | 10.00 | 10.00 | 10.00 |
| Tego™ sulfosuccinate DO 75 | | 6.67 | 6.67 |
| Propylene glycol | | 1.33 | 1.33 |
| Hexylene Glycol | | 8.00 | 8.00 |
| Heptyl Glucoside | | 6.92 | 2.72 |
| Ethanol | 70.00 | 45.12 | 45.12 |
| Water | 20.00 | 21.96 | 26.16 |

Table 9

| | T0 | T0 + 15 min | | | T0 + 480 min | | | T0 +1440 min | | |
|---|---|---|---|---|---|---|---|---|---|---|
| Ingredients | | EdT2 | C5 | C6 | EdT2 | C5 | C6 | EdT2 | C5 | C6 |
| Limonene | 100 | 1.33 | 11.32 | 6.6 | <1 | <1 | <1 | <1 | <1 | <1 |
| Ethyl Linalool | 100 | 67.48 | 80.96 | 70.8 | 9.91 | 8.4 | 6.9 | 7.26 | 2.22 | 2.6 |
| Bourgeonal™ | 100 | 81.4 | 86 | 75.6 | 43.9 | 59.8 | 43.6 | 13.42 | 28.82 | 20.9 |
| Damascone beta | 100 | 78.2 | 81.9 | 74.8 | 10.6 | 20.5 | 14.7 | 4.25 | 2.12 | 1.9 |
| Undecavertol™ | 100 | 75.7 | 84.6 | 73.2 | 8.41 | 19.1 | 14 | 4.93 | 2.62 | 3.1 |
| Verdox™ | 100 | 61.7 | 75.1 | 66.4 | 3.7 | 1.9 | 1.7 | 2.90 | <1 | 1.0 |
| Methyl ionone gamma | 100 | 79.2 | 84.0 | 74.2 | 16.4 | 32.8 | 23.3 | 3.77 | 5.68 | 5.2 |
| Cyclamen aldehyde | 100 | 81.5 | 85.8 | 75.4 | 30.7 | 45.2 | 33.2 | 7.12 | 14.71 | 12.0 |
| Ambroxan™ | 100 | 81.5 | 85.4 | 75.6 | 56.6 | 68.9 | 52.6 | 15.42 | 40.11 | 29.9 |

It can be seen from the above table that in the compositions of the invention the evaporation of fragrance ingredients is slowed down such that top note ingredients are retained up to 1h, heart note ingredients are retained up to 8h, and bottom note ingredients are retained up to 24h.

**Example 4**

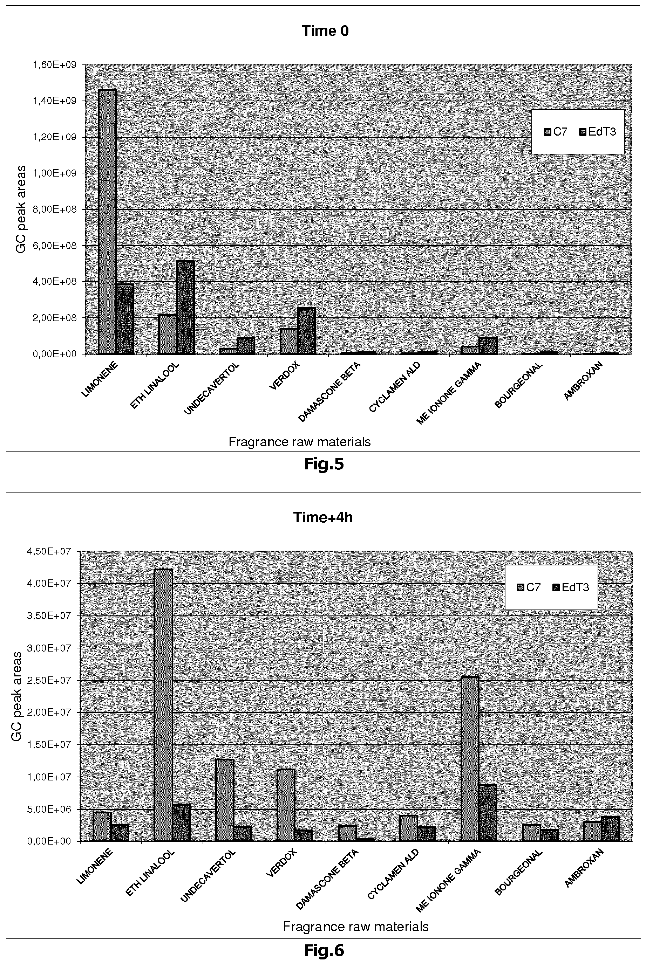

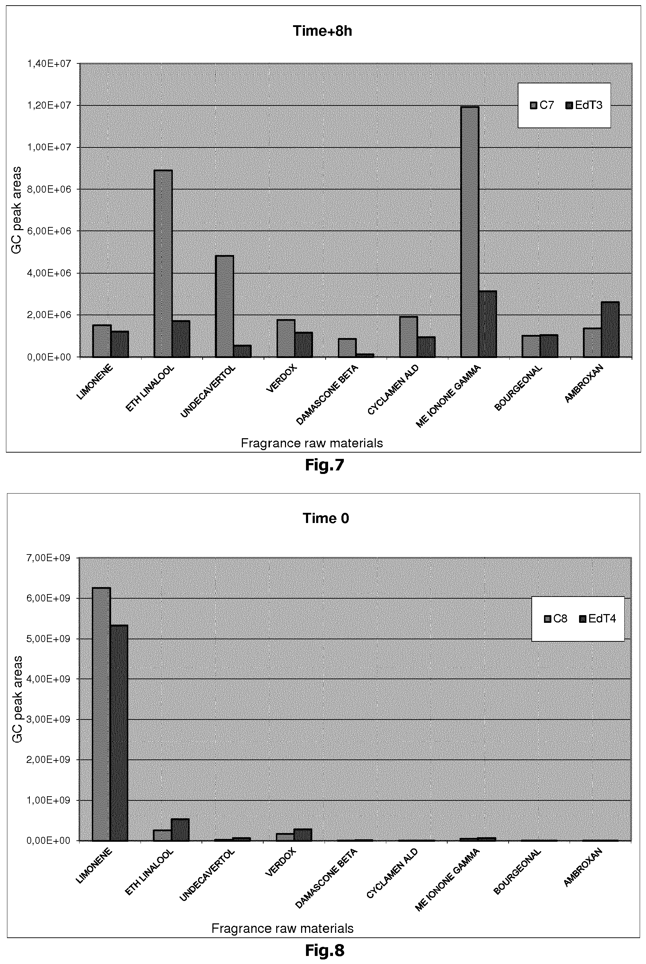

Following the procedure of example 2, the headspace concentration of representative ingredients of fragrance COM1 was determined. COM1 was dosed at 5 and 20 % either in control EdT3 and EdT4 or in compositions of the invention C7 and C8, as shown in table 10. The results are shown in figures 5 to 10.

Table 10

| Ingredients | EdT3 | EdT4 | C7 | C8 |
|---|---|---|---|---|
| Fragrance COM1 | 5.00 | 20.00 | 5.00 | 20.00 |
| Sodium Dioctylsulfosuccinate | | | 5.00 | 5.00 |
| Propylene glycol | | | 2.00 | 2.00 |
| Hexylene Glycol | | | 8.00 | 8.00 |
| Heptyl Glucoside | | | 6.92 | 6.92 |
| Ethanol | 77.50 | 77.50 | 53.08 | 38.08 |
| Water | 17.50 | 2.50 | 20.00 | 20.00 |

The following conclusions can be drawn from figures 5 to 10:

- when COM1 is dosed at 5 wt%, the headspace concentration of most of top note and heart note ingredients is higher for the compositions of the invention at T0 + 4h and at T0 + 8h;
- when COM1 is dosed at 20 wt%, the headspace concentration is significantly higher in the composition of the invention C8 for the top note ingredient ethyl linalool and for the heart note ingredient Verdox™ up to 8 hours compared to the control EdT4. After 8 hours, the heart note ingredient undecavertol is also more present in the headspace of the composition of the invention than in the control.

The results confirm that the original profile of a fragrance can be sustained for a longer period of time when the ingredients of the fragrance are formulated in a composition of the invention. The surprising effect of compositions of the invention on fragrance longevity is not related to the fragrance content and show similar benefits with different fragrance dosages.

**Example 5**

An equal weight of a commercial fragrance for women coded COM2, formulated either in control EdT1 or in composition C4 of the invention, was applied to the skin of one Caucasian female subject. The intensity of the fragrance was assessed by a panel of 14 persons trained to measure perfume intensity at time zero and after 8 hours. Perfume intensities were scored on a scale from 0 to 10 with 10 being very intense and 0 being undetectable. The results are shown in table 11.

Table 11

| Sample | T0 | T0 + 480 min* |
|---|---|---|
| EdT1 with fragrance COM 2 | 6.7 | 1.4 |
| C4 with fragrance COM 2 | 6.7 | 3.0 |
| *Statistical variance analysis with 2 factors (DUNCAN multiple comparison with judge and products effects at 95 % confidence level) | | |

It can be seen that 8 hours after application the composition of the invention was perceived with a significantly stronger intensity on skin compared to a conventional EdT.

**Example 6**

A panel of 19 people of both sexes assessed samples of fragrance COM3 (the composition of which is shown in table 12), formulated either in a conventional EdT or in a composition of the invention (EdT5 and C9, respectively, see table 13), applied to their forearms from a pump dispenser. The individuals did not know which sample was applied to which forearm and the order of application was randomised. The panelists were asked to assess the overall fragrance intensity after set times, using a 0 to 10 intensity scale as described in example 5, and also to assess the intensity of top, heart and bottom note ingredients. They were also asked to score how much they liked the fragrance at the different times and to describe the fragrance by choosing "All That Apply attributes". The results are shown in tables 16 to 19 below. An * symbol indicates a statistically significant difference at the 95% confidence level.

The panelists were asked to check all of the following attributes that applied to the fragrances they smelt:

Floral
Fruity
Citrus
Green
Musk
Woody
Pear
Peony
Jasmine
Muguet
Red Berries
Herbal
Tangerine
Moss
Praline/ Gourmand
Oriental

Table 12

| Ingredients | CAS No | wt% | Category |
|---|---|---|---|
| Hexyl acetate | 142-92-7 | 0.60 | Top |
| Triplal | 68039-49-6/144046-32-2 | 0.10 | Top |
| Hexen-1-ol, cis-3 | 928-96-1/95123-47-0 | 0.25 | Top |
| Lemon oil Italian nat EO | 8008-56-8 | 1.80 | Top |
| Orange oil pera white Brazil [FFP] | 8008-57-9 | 0.60 | Top |
| Ethyl linalool | 10339-55-6 | 3.60 | Top |
| Heliobouque™ | 1205-17-0 | 1.10 | Heart |
| Phenyl ethyl alcohol white extra | 60-12-8 | 0.10 | Heart |
| Damascone alpha | 24720-09-0 | 0.05 | Heart |
| Dynascone neat | 56973-85-4 | 0.05 | Heart |
| Undecalactone gamma (aldehyde C-14) | 104-67-6/57084-17-0 | 0.05 | Heart |
| Citronellyl acetate | 150-84-5 | 0.50 | Heart |
| Verdox™ | 88-41-5 | 0.60 | Heart |
| Geranyl acetate extra | 105-87-3 | 1.00 | Heart |
| Magnolan™ | 27606-09-3 | 1.30 | Heart |
| Laurinal™ | 107-75-5/123238-75-5 | 3.50 | Heart |
| Laurinal™ , S | 34212-53-8 | 8.00 | Heart |
| Lilial™ | 80-54-639390-70-0 | 4.00 | Heart |
| Oakmoss | 9000-50-4 | 0.20 | Bottom |
| Hedione™ | 24851-98-7/128087-96-7 | 30.00 | Bottom |
| Orbitone™ | 54464-57-2/166090-45-5 | 10.00 | Bottom |
| Galaxolide™ , 50% in DPG* | 1222-05-5 | 28.80 | Bottom |

(continued)

| Ingredients | CAS No | wt% | Category |
|---|---|---|---|
| Ambroxan™ | 6790-58-5 | 0.80 | Bottom |
| Boisambrene™ forte | 58567-11-6 | 1.20 | Bottom |
| Cassis base 345B | NA | 1.80 | NA |

\* dipropylene glycol
Heliobouquet™ is 3-(1,3-benzodioxol-5-yl)-2-methylpropanal; Laurinal™ is (3R)-7-Hydroxy-3,7-dimethyl-octanal; Lilial™ is 3-(4-*tert*-Butylphenyl)-2-methylpropanal; Galaxolide™ is 1,3,4,6,7,8-hexahydro-4,6,6,7,8,8-hexamethylcyclopenta(g)-2-benzopyran; Boisambrene™ is ethoxymethoxycyclododecane

Table 13

| Ingredients | EdT5 | C9 |
|---|---|---|
| Fragrance COM3 | 15.0 | 15.0 |
| Sodium Dioctylsulfosuccinate | | 5.0 |
| Propylene glycol | | 2.0 |
| Hexylene Glycol | | 8.0 |
| Heptyl Glucoside | | 6.9 |
| Ethanol | 77.5 | 45.1 |
| Water | 7.5 | 18.0 |

Table 14: Global Intensity Assessment

| Sample | T0 | T0 + 120 min | T0 + 240 min | T0 + 360 min* |
|---|---|---|---|---|
| EdT5 | 6.8 | 5.7 | 4.2 | 3.3 |
| C9 | 6.6 | 6.0 | 4.9 | 4.3 |

Table 15: Top Note Ingredients Intensity Assessment

| Sample | T0 | T0 + 120 min* | T0 + 240 min* | T0 + 360 min* |
|---|---|---|---|---|
| EdT5 | 6.5 | 4.5 | 2.7 | 1.8 |
| C9 | 6.5 | 5.3 | 3.8 | 2.9 |

Table 16: Heart Note Ingredients Intensity Assessment

| Sample | T0 | T0 + 120 min | T0 + 240 min | T0 + 360 min* |
|---|---|---|---|---|
| EdT5 | 5.8 | 5.3 | 4.0 | 2.9 |
| C9 | 5.6 | 5.5 | 4.5 | 3.8 |

Table 17: Bottom Note Ingredients Intensity Assessment

| Sample | T0 | T0 + 120 min | T0 + 240 min | T0 +360 min |
|---|---|---|---|---|
| EdT5 | 4.9 | 4.9 | 4.0 | 3.4 |
| C9 | 4.6 | 4.4 | 4.4 | 3.8 |

The results indicate stronger intensities of the top and heart note ingredients over time. The overall intensity of the composition of the invention was rated significantly higher than that of the control EdT at the 6 hour time point, at 95% confidence. The intensity of the composition of the invention was also rated higher at the 4 hour time point.

The intensity of the composition of the invention was also rated significantly higher than that of the control EdT for Top Note ingredients at the 2 hour, 4 hour and 6 hour time points, at 95% confidence.

The intensity of the composition of the invention was also rated significantly higher than that of the control EdT for heart note ingredients at the 6 hour time point, at 95% confidence.

Finally, the intensity of the composition of the invention was comparable to that of the control EdT for bottom note ingredients at all time points.

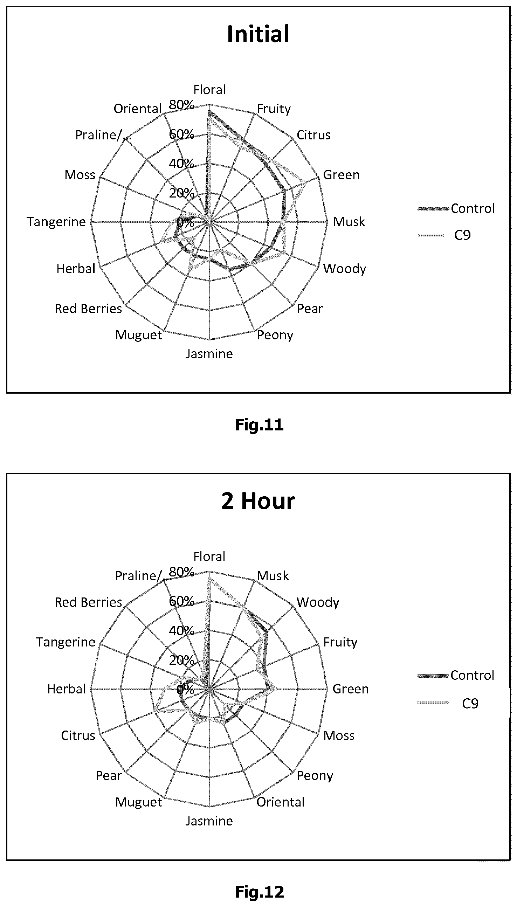

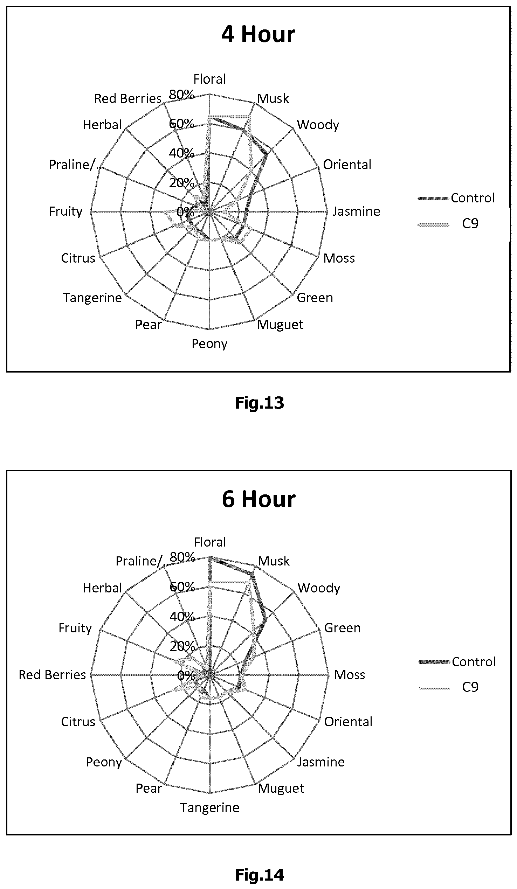

Figures 11 to 14 show the olfactive mapping of fragrance COM3 per attribute and over time. In the charts the length of each attribute corresponds to the number of panelists applying said attribute to the fragrance. Initially, the control EdT was identified as higher in Peony while the composition of the invention was more Green. At 2 hours Citrus notes are called out for the composition of the invention. At 4 hours the control EdT was more Woody, Jasmine and the composition of the invention was higher in Musk and Fruity notes. At 6 hours, the control EdT was mostly Floral, Musk and the composition of the invention pushed slightly more Citrus, Fruity notes.

**Example 7**

Fragrance compositions were prepared and assessed for limpidity by eye. The results are shown in table 18.

Table 18

| Ingredients | C10 | C11 | C12 | C13 |
|---|---|---|---|---|
| Fragrance COM1 | 10.0 | 10.0 | 10.0 | 10.0 |
| Sodium Dioctyl sulfosuccinate | 5.0 | 5.0 | - | - |
| Sulfopon™ 1216G* | | | 6.5 | |
| Glucotain™ Care** | | | | 16.25 |
| Propylene glycol | 2.0 | 2.0 | 1.3 | 1.3 |
| Hexylene Glycol | 8.0 | 8.0 | 7.7 | 7.7 |
| Tween™ 20 | 9.9 | - | - | - |
| Sisterna™ L70C*** | | 9.9 | - | - |
| Heptyl Glucoside | | | 6.9 | 6.9 |
| Water | 40.1 | 40.1 | 19.0 | 19.0 |
| Ethanol | 25.0 | 25.0 | 48.6 | 38.85 |
| | | | | |
| Limpidity | Yes | Yes | Yes | Yes |
| * Sodium Coco-Sulfate<br>**Capryloyl/Caproyl Methyl Glucamide (40% active matter)<br>***Sucrose laurate | | | | |

Example 7 shows that various surfactants can be used in the composition of the present invention with equal effect on the desired limpidity and long lasting property of the fragrance (data not shown for the latter property).

**Example 8**

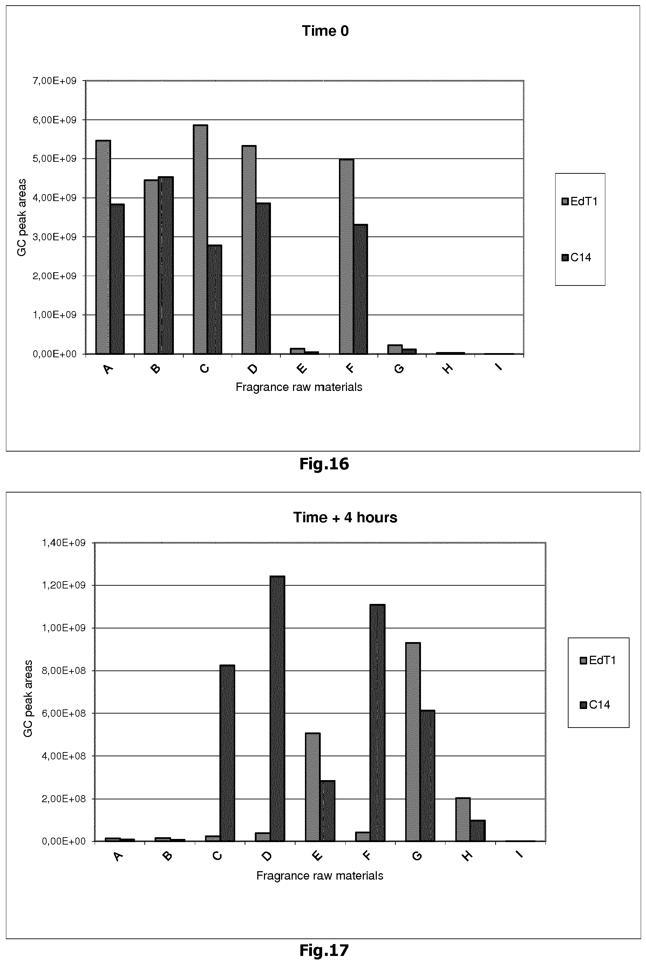

The headspace concentration of individual ingredients of fragrance TAK (see Table 1), as formulated in either a conventional EdT (EdT1) or fragrance compositions of the invention (C14, C15), was assessed by the method described in example 2. The results are shown in figures 16 to 21. The fragrance ingredients are labeled by letters as indicated in Table 1.

Table 19

| Ingredients | EdT1 | C14 | C15 |
|---|---|---|---|
| Fragrance TAK | 10.0 | 10.00 | 10.00 |
| Aerosol™ OT 100 | - | 14.88 | - |
| Propylene glycol | - | 2.00 | 2.00 |
| Hexylene Glycol | - | 8.00 | 8.00 |
| Tween ™ 20 | - | - | 14.88 |
| Ethanol | 65.00 | 45.12 | 25.12 |
| Water | 25.00 | 20.00 | 40.00 |

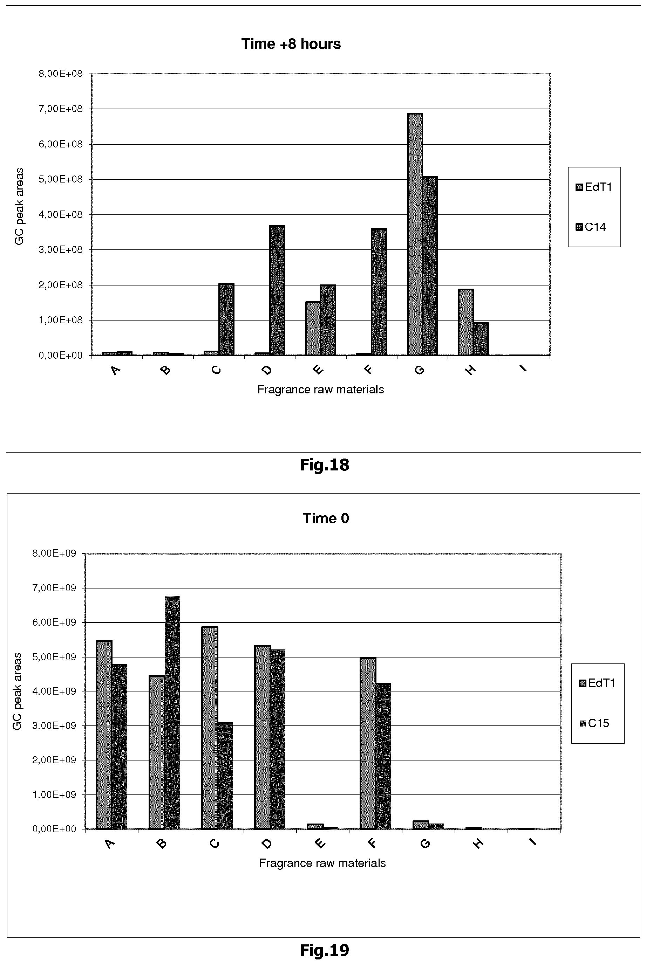

Figures 16 and 19 show that the headspace concentration of the control EdT1 is somewhat higher than that of compositions C14 and C15 at TO (for all fragrance ingredients).

Figures 17 and 18 show that the headspace concentration measured for top note ingredient linalool and for heart note ingredients linalyl acetate and Verdox™ is significantly greater at T0+4h and T0+8h in composition C14 (compared to control EdT1).

Figure 18 further shows that the headspace concentration measured for heart note ingredient geraniol is somewhat greater at T0+8h in composition C14 (compared to control EdT1).

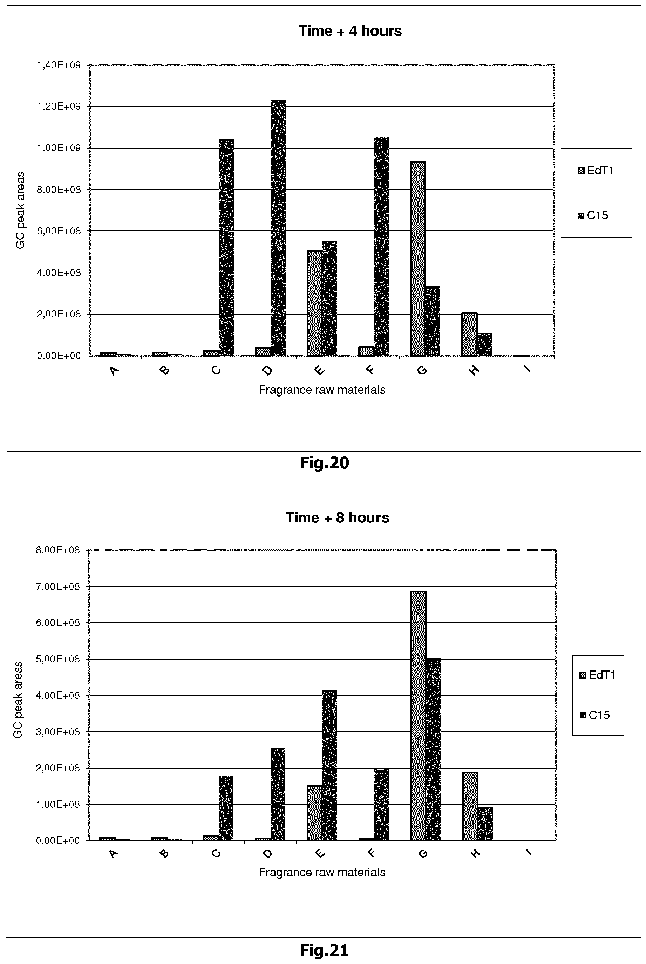

Figures 20 and 21 show that the headspace concentration measured for top note ingredient linalool and for heart note ingredients linalyl acetate, geraniol and Verdox™ is greater at T0+4h and T0+8h in composition C15 (compared to control EdT1), the difference being significant for linalool, linalyl acetate and Verdox™.

**Claims**

1. A fragrance composition comprising, by weight of the composition:

    a) about 1.0 to about 30.0 wt% fragrance;
    b) about 2.0 to about 30.0 wt % of a system comprised of a diol and a surfactant;
    c) about 25.0 to about 80.0 wt% ethanol;
    d) at least about 5.0 wt% water;

    wherein the fragrance and the system (diol + surfactant) together make up at most 60.0 wt% of the composition; wherein water and ethanol together make up at most 97.0 wt% of the composition; wherein the surfactant is not a phospholipid.

2. The fragrance composition of claim 1, which comprises from about 1.0 to about 25.0 wt% of fragrance.

3. The fragrance composition of claim 1 or claim 2, which comprises from about 5.0 to about 25.0 wt% of a system comprised of a diol and a surfactant.

4. The fragrance composition of any one of claims 1 to 3, wherein the diol is selected from vicinal diols, non-vicinal diols and mixtures thereof.

5. The fragrance composition of claim 4, wherein the diol is a non-vicinal diol or a mixture of non-vicinal diols.

6. The fragrance composition of any one of claims 1 to 5, wherein the system:fragrance weight ratio is in the range from about 5:1 to about 0.8:1, preferably in the range from about 3.5:1 to about 0.8:1.

7. The fragrance composition of any one of claims 1 to 6, wherein the surfactant is selected from anionic surfactants, cationic surfactants, zwitterionic surfactants, amphoteric surfactants, non-ionic surfactants and mixtures thereof.

8. The fragrance composition of claim 7, wherein the surfactant is selected from anionic surfactants, non-ionic surfactants and mixtures thereof.

9. The fragrance composition of claim 8, wherein the surfactant comprises a mixture of anionic surfactant(s) and non-ionic surfactant(s).

10. The fragrance composition of any of claims 7 to 9, wherein the anionic surfactant is selected from dialkyl glutamates, dialkyl phosphates, dialkyl sulphosuccinates, and mixtures thereof.

11. The fragrance composition of any of claims 7 to 10, wherein the non-ionic surfactant is selected from sorbitan esters with lauric, oleic, myristic, stearic, or palmitic acid; sucrose esters with $C_8$-$C_{20}$ fatty acid; polyglyceryl esters comprising from 1 to 10 glycerin units and one or two $C_4$ to $C_{20}$ aliphatic alkyl groups; $(C_6$-$C_{16})$alkyl glucosides; glucamides; and mixtures thereof.

12. The fragrance composition of any one of claims 9 to 11, wherein the weight ratio of non-ionic surfactant(s) to anionic surfactant(s) is in the range from about 3:1 to about 1:3, preferably in the range from about 2:1 to about 1:1.5.

13. The fragrance composition of any one of claims 1 to 12, wherein the surfactant:fragrance weight ratio is in the range from about 5:1 to about 0.5:1.

14. The fragrance composition of any one of claims 1 to 13, which is a fine fragrance composition, preferably in the form of a perfume concentrate, a perfume, an eau de toilette, an eau de parfum, a cologne or a body mist.

15. The fragrance composition of any one of claims 1 to 13, which is in the form of a spray.

Fig.1

Fig.2

Fig.3

Fig.4

Fig.5

Fig.6

**Fig.7**

**Fig.8**

**Fig.9**

**Fig.10**

## Initial

**Fig.11**

## 2 Hour

**Fig.12**

# 4 Hour

**Fig.13**

# 6 Hour

**Fig.14**

**Fig.15A**

**Fig.15B**

**Fig.15C**

**Fig.16**

**Fig.17**

**Fig.18**

**Fig.19**

**Fig.20**

**Fig.21**

Europäisches
Patentamt

European
Patent Office

Office européen
des brevets

# EUROPEAN SEARCH REPORT

Application Number

EP 18 30 5289

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JP H08 134491 A (POLA CHEM IND INC) 28 May 1996 (1996-05-28) | 1-8,13, 14 | INV. A61K8/34 A61Q13/00 |
| Y | * example 1 * <br> * tables 1,4 * <br> * claims * | 1-15 | |
| Y | CN 101 032 454 A (ZHOU RONG [CN]) 12 September 2007 (2007-09-12) * the whole document * | 1-15 | |
| A | EP 2 340 804 A1 (TAKASAGO PERFUMERY CO LTD [JP]) 6 July 2011 (2011-07-06) * claims; examples * | 1-15 | |
| A | WO 2013/060691 A2 (FIRMENICH & CIE [CH]) 2 May 2013 (2013-05-02) * claims 1-12 * | 1-15 | |
| Y | US 2014/162932 A1 (HATAKEYAMA TADAHIDE [JP] ET AL) 12 June 2014 (2014-06-12) * claims; examples * | 1-15 | TECHNICAL FIELDS SEARCHED (IPC) <br> A61K <br> A61Q |

The present search report has been drawn up for all claims

| Place of search | Date of completion of the search | Examiner |
|---|---|---|
| Munich | 30 April 2018 | Cismaru, L |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 18 30 5289

This annex lists the patent family members relating to the patent documents cited in the above-mentioned European search report.
The members are as contained in the European Patent Office EDP file on
The European Patent Office is in no way liable for these particulars which are merely given for the purpose of information.

30-04-2018

| Patent document cited in search report | | Publication date | Patent family member(s) | | Publication date |
|---|---|---|---|---|---|
| JP H08134491 | A | 28-05-1996 | NONE | | |
| CN 101032454 | A | 12-09-2007 | NONE | | |
| EP 2340804 | A1 | 06-07-2011 | EP | 2340804 A1 | 06-07-2011 |
| | | | JP | 2011136995 A | 14-07-2011 |
| | | | US | 2011177995 A1 | 21-07-2011 |
| WO 2013060691 | A2 | 02-05-2013 | BR | 112014009848 A2 | 18-04-2017 |
| | | | CN | 104114147 A | 22-10-2014 |
| | | | CO | 6950485 A2 | 20-05-2014 |
| | | | EP | 2771071 A2 | 03-09-2014 |
| | | | JP | 6072050 B2 | 01-02-2017 |
| | | | JP | 2015501311 A | 15-01-2015 |
| | | | MX | 343010 B | 21-10-2016 |
| | | | RU | 2014121234 A | 10-12-2015 |
| | | | US | 2015031596 A1 | 29-01-2015 |
| | | | WO | 2013060691 A2 | 02-05-2013 |
| US 2014162932 | A1 | 12-06-2014 | JP | WO2013015103 A1 | 23-02-2015 |
| | | | US | 2014162932 A1 | 12-06-2014 |
| | | | WO | 2013015103 A1 | 31-01-2013 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4264478 A
- US 2383517 A
- US 6172037 B
- EP 181401 A
- WO 2005070371 A
- US 5104646 A
- US 5106609 A

**Non-patent literature cited in the description**

- **ERNEST GUENTHER.** The Essential Oils. D. Van Nostrand, 1948
- Perfume Flavors and Chemicals. **S. ARCTANDER.** the Merck Index. vol. I and II
- McCutcheon's Surfactants and Detergents. North American & International Editions
- Fragrance Chemistry - The Science of the Sense of Smell. Academic Press, 1982